# EUROPEAN PATENT APPLICATION

(11) **EP 1 840 573 A1**
(43) Date of publication of application: **03.10.2007**
(21) Application number: 06290485.9
(22) Date of filing: 27.03.2006
(51) Int. Cl.: G01N 33/564

(54) **Secreted proteins as early markers and drug targets for autoimmunity, tumorigenesis and infections**

(71) Applicant: INSTITUT PASTEUR, 75724 Paris Cédex 15 (FR)
(72) Inventor: Melanitou-McClymont, Evie, 75004 Paris (FR)
(74) Representative: Sellin, Carole

(57) **Abstract**

The present invention relates to a method for the early diagnosis of a disease having a pre-inflammatory phase, or for the diagnosis of a predisposition to said disease, in a mammal, prior to any clinical signs, comprising measuring the level of at least a marker protein chosen amongst the following murine proteins Pap, Reg3a, Reg2, Cel, Reg1, Tff2, Clps, Spp1, Klk9, Klk6, Rib1, Klk5, Muc1, Cckar, Ggh, Ang, Nucb2, Pnliprp2, Pla2g1b, Ela1, Ela2, 2210010C04Rik, Pnliprp1, Itmap1, Vtn, C1qb, Sycn, Amy1, Ctrb1, 1110002023Rik, 1810014L12Rik and their respective mammalian orthologs, in a body fluid or tissue sample obtained from said mammal, comparing the measured level to a reference level for said marker protein and diagnosing the later onset of said disease if the measured level is significantly superior to the reference level. The invention also encompasses related methods and uses of these early markers of diseases having a pre-inflammatory phase. A preferred mammal is human and a preferred diseases are auto-immune diseases, especially type 1 diabetes.

## Description

The present invention is in the domain of diagnosis, especially in the domain of human diagnosis, of autoimmune diseases, cancers and infections.

Epidemiological data have shown that the incidence of autoimmune diseases is increasing in industrialized countries. In particular, type 1 diabetes (T1D) shows a north-south gradient, with the highest incidence in northern Europe (1% to 1.5% of the population in Finland) and decreasing incidence in more southerly and tropical locations.

Autoimmune diabetes can occur at any age, but is most commonly diagnosed in childhood. In humans it is characterized by absolute insulin-dependency and the association of disease with the presence of certain alleles of the Human Leukocyte Antigen (HLA) class II genes, within the major histocompatibility complex (MHC). The autoimmune etiology is characterized by infiltration of the islets of Langerhans by immune cells (insulitis) and serum autoantibodies {Bottazzo, 1986}.

The genetic transmission of T1D corresponds to a multigenic and multifactorial inheritance, which is characteristic of complex characters where environmental factors play a role. Population based studies have suggested that T1D in monozygotic (MZ) twins shows a cumulative risk from birth to age 35 years reaching 70%. This degree of familial clustering is consistent with a significant genetic input to the disease. However the level of discordance indicates that susceptibility genes have incomplete penetrance, i.e. an individual who is genetically programmed for T1D does not always develop the clinical condition. This latter observation shows that environmental factors influence the genetic component of the disease.

Interestingly a high percentage of subjects with T1D have also other autoimmune diseases. Fifteen to 30% of diabetics develop also thyroid disease, 4-9% develops celiac disease and 0.5% has Addison's disease {Reviewed by Barker, 2006}. Similarly in the NOD mouse, the most well studied animal model for T1D, several other autoimmune diseases occur {Ridgway, 1996}. Common genetic risk factors have been associated and the presence of genes playing a common role has been proposed {Reviewed by Barker, 2006}.

Although numerous reports have been published, in human and animal models, genetic approaches have been somewhat limited in the discovery of autoimmune diabetes genes. Many questions still remain as to why immunoregulatory mechanisms and self tolerance break down, why antigens normally ignored by the immune system seem so provocative to the lymphocytes of diabetic individuals.

Animal models have proven very useful in studies centered on deciphering the etiology of the disease, and also for elaborating protocols for clinical trials. The difficulty to obtain human tissues, pancreas for autoimmune diabetes, emphasises the importance of the existent animal models in the study of the disease pathogenesis. In this respect, the NOD (Non-Obese Diabetic) mouse is a widely studied animal model for autoimmune diseases. Indeed, there are close clinical and immunological similarities to the human type 1 diabetes, and without insulin treatment, the animals die within two months. The pathophysiological dissection of type 1 diabetes in the NOD mouse has allowed this disease to be one of the best examples in the study of complex characters.

Although the NOD mouse is clearly not completely analogous to human disease, the study of the pathogenesis of diabetes in this model allows parallels to be made and insights to be drawn which ultimately help in understanding the development of diabetes in humans.

An additional hindrance in gene discovery is that the low penetrance of genes implicated in autoimmune diseases renders their identification difficult. A better understanding of the phenotypic characteristics of the disease, as well as the possibility to apply experimental "perturbations" in the pathological processes involved, might bypass these difficulties and help gene discovery. Such "perturbations" might be environmental triggers of the immune system, for example infections, known to influence the disease.

Recessive, dominant and multifactorial hypotheses have been advanced in the past, however, it is now commonly accepted that environmental factors are also involved. It has been suggested that the rise in the incidence of T1D worldwide may be due to environmental influences such as viruses. Viruses have been considered as an important factor triggering T1D in genetically susceptible humans and in many animal models. The mechanism of viral infection may be due to its direct or indirect effect on the β cells of the pancreas or directly on the lymphoid cells.

Rotavirus for example, has been suspected as the cause of a clinically silent pancreatic infection in patients with rapid disease onset that may have led to a rapid T cell mediated loss of β cells in the islets. Rotaviruses can infect islets in tissue culture, but these viruses are not infectious unless they are activated by trypsin secreted by the exocrine pancreas.

Furthermore, islet β cells produce a wide range of antiviral responses for host protection that may stimulate immune mediated islet destruction. There is accumulating evidence that viral induction of the cytokine, interferon alpha (IFNα), is strongly implicated in the disease process. Induction of autoimmunity has also been demonstrated by an IFNα induced HERV-K18 super antigen expression, that activates auto reactive T cells. In addition, IFNα can accelerate and induce autoimmune diabetes when the B7.1 human co-stimulatory molecule, under the rat insulin promoter, is expressed as a transgene in islets, in mice. These observations have to be taken in consideration when gene discovery approaches are undertaken.

The present invention is based on the identification of genes implicated in the early steps of autoimmune destruction. These genes are also of interest in order to unravel the mechanisms responsible for the autoimmune condition. It is proposed that genes involved at an early stage might be responsible for immune events leading to the inflammatory autoimmune response and might also respond to environmental factors such as infections.

The inventor has established a first early phenotypic marker {Melanitou, 2004, 2005}, early insulin auto-antibody, allowing to select autoimmune prone individual mice and to perform a systematic differential gene expression search by high throughput analysis of the transcriptome. The data obtained by these experiments have demonstrated the existence of genes that are differentially regulated at an early stage, prior to clinical signs, among disease prone animals, in comparison with non disease prone individuals.

The inventor has now identified these differentially expressed genes. These genes seem to be earlier markers of a disease-prone state than the previously known markers and they are specific to the pre-inflammatory stage of the disease. Whereas insulin auto-antibodies reflect an immune state of the patient, the markers now identified by the inventor have the potential to reflect the molecular mechanisms initiating the pre-inflammatory process.

Among the genes identified, 32 of them code for proteins located in the extra cellular space or for proteins which, according to functional analysis, are potentially secreted. They present the potential to be used as diagnostic biomarkers for type 1 diabetes or other autoimmune diseases during the pre-inflammatory stages. Due to their early differential expression they have also a potential role as therapeutic targets for autoimmunity and other diseases that are preceded by a pre-inflammatory stage (certain cancers and infections for example).

Secreted proteins have indeed properties that lend themselves to use as therapeutic agents or targets. They are accessible to various drug delivery mechanisms, because they are within the extracellular space.

This invention concerns 32 genes coding for proteins located in the extracellular space or proteins potentially secreted and suitable for use as diagnostic biomarkers for type 1 diabetes or other autoimmune diseases during the pre-inflammatory stages. These proteins are also suitable therapeutic targets since the corresponding coding genes are differentially expressed in early pre-inflammatory stages prior to autoimmune destruction observed in type 1 diabetes. Some of these genes are also implicated in certain cancers and infectious diseases at the pre-inflammatory stages.

The present invention relates to an in vitro method for the early diagnosis of a disease having a pre-inflammatory phase, in a mammal, prior to any clinical signs in connection with said disease. The method comprises the steps of:
a) measuring the level of a marker in a body fluid or a tissue sample obtained from said mammal,
b) comparing the measured level to a reference level for said marker, and
c) diagnosing the later onset of said disease if the measured level is significantly divergent from the reference level.

The invention also relates to an in vitro method for diagnosing a disease-prone state in a mammal, prior to any clinical signs of said disease, wherein the disease has a pre-inflammatory phase. A disease-prone state reflects a risk of developing the disease. Said method comprises the steps of:
a) measuring the level of a marker in a body fluid or a tissue sample obtained from said mammal,
b) comparing the measured level to a reference level for said marker, and
c) diagnosing a disease-prone state if the measured level is significantly divergent from the reference level.

By "divergent", it is meant that the measured level is greater than or less than the reference level. By "significantly divergent", it is to be understood that the difference between the measured level and the reference level is significant from a statistical point of view, that is the difference is greater that the standard deviation of the measured levels. The difference is thus preferably greater that the error inherent in the measurement and greater than the variations observed between individuals, independently of the disease.

The marker used in the diagnostic methods of the invention is preferably a marker protein, which is chosen amongst the following murine proteins, encoded by the 32 genes of the invention:
- Pap (pancreatic associated protein, encoded by AV371861),
- Pap (pancreatic associated protein, encoded by D63359),
- Reg3a (Regenerating protein, encoded by D63357),
- Reg2 (encoded by D14011),
- Cel (carboxyl ester lipase encoded by U37386),
- Reg1 (encoded by D14010),
- Tff2 (trefoil factor 2, also known as SP; mSP (spasmolytic protein 1), encoded by U78770),
- Clps (colipase, pancreatic, encoded by AA710635),
- Spp1 (osteopontin, encoded by X13986),
- Klk9 (kallikrein 9 encoded by M17962),
- Klk6 (kallikrein 6 also known as Kal; Klk1, mGk-6 and 0610007D04Rik, encoded by M13500),
- Rib1 (pancreatic ribonuclease also known as Rib-1 and AI574248, encoded by X60103),
- Klk5 (kallikrein 5 or mGK-5, encoded by Y00500),
- Muc1 (also known as Mucin or episialin, encoded by M84683),
- Cckar (Cholecystkinin receptor, encoded by D85605),
- Ggh (gamma glutamyl hydrolase, encoded by AF051102),
- Ang (angiogenin, encoded by U22516),
- Nucb2 (nucleobindin 2, encoded by AJ222586),
- Pnliprp2 (pancreatic lipase-related protein 2, encoded by M30687),
- Pla2g1b (phospholipase A2, group IB, pancreas, encoded by Al327450),
- Ela1 (elastase 1, encoded by M27347),
- Ela2 (elastase 2, encoded by X04573),
- 2210010C04Rik (encoded by AE000663),
- Pnliprp1 (pancreatic lipase related protein 1, encoded by AA674409),
- Itmap1 (integral membrane-associated protein 1; having CUB and zona pellucida-like domains 1, encoded by AV059956),
- Vtn (vitronectin, encoded by M77123),
- C1qb (encoded by AV367855),
- Sycn (syncollin, encoded by AA607809),
- Amy1 (amylase 1, salivary, encoded by J00356),
- Ctrb1 (chymotrypsinogen B1, also known as 2200008D09Rik; encoded by AA590358),
- 1110002023Rik (encoded by AW122851) and
- 1810014L12Rik (encoded by AI852985),
and their corresponding mammalian orthologs in the diagnosed mammal.

Particularly preferred markers are chosen amongst the following murine proteins: Pap, Reg3a, Reg2, Cel, Reg1, Tff2, Clps, Spp1, Klk9, Klk6, Rib1, Klk5, Muc1, Cckar, Pnliprp2, Pla2g1b, Ela1, Ela2, 2210010C04Rik, Pnliprp1, Itmap1, Sycn, Amy1 and Ctrb1, and their respective mammalian orthologs.

Alternatively, the marker chosen may also be the DNA or RNA transcript corresponding to one of these proteins.

Ortholog genes are defined as genes in different species that evolved from a common ancestral gene by speciation. They can be traced evolutionarily through different species. By comparing the ortholog sequences of a specific gene between many species, the amino acid sequences which are conserved can be determined. These highly conserved sequences provide information on which amino acids are essential to the protein structure and function. Normally, orthologs retain the same function in the course of evolution.

According to a preferred embodiment of the present invention, the measured level of the marker is superior to the level of the reference. The difference between the measured level and reference level may significantly vary depending on the marker, the level in a disease-prone individual is however greater than the limits of detection, and above the cut-off line. For some of the markers, the measured level may be for example at least 5% superior to the reference level, preferably at least 10% or 20% superior, depending on the reference level.

In the embodiment wherein the chosen marker is RNA, the measured level is at least 3 times the reference level, generally at least 6 times, and in the majority of the cases at least 10 times higher than the reference level.

The reference level of a marker in a body fluid or tissue of a given mammal is the mean level of said marker in the same fluid or tissue, measured in healthy individuals having no known predisposition to the disease.

The reference level of a RNA or DNA marker in a body fluid or a tissue is also measured in healthy individuals having no known predisposition to the disease.

The level of a protein may be expressed as a concentration of said protein in a body fluid or a tissue; it may also be expressed as a relative abundance of said protein, with respect to another compound of the fluid or tissue. The level of the protein may for example be expressed as a number of mole per mole of albumin (especially for expressing the level of a marker in peripheral blood), or as a number of mole per mole of creatinin (especially for expressing the level of a marker in urine). These different potential units are well known to the skilled person in the domain of diagnostic methods.

For some of the markers however, the sole detection of the marker in a body fluid or a tissue (that is detection of a level which is above the cut-off line) is indicative of a disease-prone state or indicative of a disease, without comparison of this level to a reference level. Indeed, some of these markers are normally not expressed; therefore, their sole presence in a body fluid (or in a tissue) is an indication of a pre-inflammatory stage. Such markers are for example Pap, Reg3a, Cckar, Ang and Spp1 and their respective mammalian orthologs, especially their human orthologs.

Alternatively, the methods of the invention also encompass the detection in a body fluid of a mammal to be diagnosed, of auto-antibody directed against one of the following murine proteins: Pap, Reg3a, Reg2, Cel, Reg1, Tff2, Clps, Spp1, Klk9, Klk6, Rib1, Klk5, Muc1, Cckar, Ggh, Ang, Nucb2, Pnliprp2, PIa2g1b, Ela1, Ela2, 2210010C04Rik, Pnliprp1, Itmap1, Vtn, C1qb, Sycn, Amy1, Ctrb1, 1110002O23Rik, 1810014L12Rik or their respective mammalian orthologs. Such auto-antibodies are generally absent in healthy mammals. Example 5 illustrates this embodiment. With regard to the disease to be diagnosed by the methods of the invention, it is well known that different diseases, with different aetiologies, possess a pre-inflammatory stage. This pre-inflammatory stage is generally exempt of any clinical symptom or early signs of the disease. Therefore, at the time the methods are carried out, there is no detectable sign of the disease. A predisposition to a disease may however be suspected, especially for individuals of families comprising members suffering from said disease or for individuals living in an environment likely to be favourable to the development of the disease. Preferred mammals to be diagnosed according to the methods of the invention are mammal suspected of a predisposition to the disease. The predisposition may also be suspected from a genetic analysis.

Autoimmune diseases are characterized by a pre-inflammatory phase, these diseases are thus particularly preferred in the context of the present invention. Examples of such diseases which can advantageously be diagnosed early by the methods of the invention are *inter alia* type 1 diabetes mellitus, multiple sclerosis, rheumatoid arthritis, collagen induced arthritis and autoimmune hepatitis. A predisposition to one of these diseases may also be diagnosed or prognosed according to the methods of the invention.

For diagnosis of type 1 diabetes, particularly preferred markers to be used in the methods of the invention are proteins chosen in the group consisting of Reg 1, Reg2, Cel, Cckar and Spp1 and their mammalian orthologs, especially their human orthologs: Lithostathine (REG1A), Regenerating protein I beta, Carboxyl ester lipase (bile salt-stimulated lipase) (CEL), Cholecystokinin A receptor and Osteopontin (OPN).

For diagnosis of multiple sclerosis, a particularly preferred marker to be used in the methods of the invention is Spp1 or its mammal orthologs, especially its human ortholog Osteopontin (OPN).

For diagnosis of collagen induced arthritis, a particularly preferred marker to be used in the methods of the invention is Spp1 or its mammal orthologs, especially its human ortholog Osteopontin (OPN).

For diagnosis of collagen autoimmune hepatitis, a particularly preferred marker to be used in the methods of the invention is Spp1 or its mammal orthologs, especially its human ortholog Osteopontin (OPN).

Type 1 diabetes mellitus is a particularly preferred embodiment of the diagnostic methods of the invention. According to said methods, it is indeed possible to define the propensity to the further onset of type 1 diabetes before any clinical sign of insulitis or pre-insulitis. Therefore, this disease may be diagnosed very early according to the invention and thus the treatment can be undertaken before any damage of the islet β cells of the patient. This early treatment delays the onset of the disease and improves the quality of life of the patient.

In accordance with this embodiment, the diagnostic methods of the invention may also be carried out in association with the detection of the presence of insulin auto-antibodies in the peripheral blood of the diagnosed mammal. Indeed, the methods of the invention may be combined with the detection of said auto-antibody. Alternatively, it is also envisaged that the mammal to be diagnosed is primarily tested for the presence of insulin auto-antibody before being subjected to the diagnostic methods of the invention. In this case, the mammals subjected to a method of the invention are preferably mammals which have been previously been tested as positive for the presence of insulin auto-antibody. They can alternatively be negative for insulin auto-antibody.

Another possibility is to carry out the detection of the presence of insulin auto-antibody after having carried out the diagnostic methods of the invention. The markers of the invention indeed appear as earlier markers of the disease than insulin auto-antibodies. Moreover, the markers of the invention are capable of distinguishing the beginning of a pre-inflammatory phase in individuals who are negative for the presence of insulin auto-antibodies.

Other diseases having a pre-inflammatory stage and encompassed by the present invention are certain cancers. The pre-inflammatory phase corresponds to the time of possible tissue disorganization, in certain cancers, such as for example pancreatic cancer, breast cancer, renal cancer, colorectal cancer, ovarian cancer and gastric cancers. The pre-inflammatory stage of these cancers is usually difficult to detect, rendering the diagnostic methods of the invention particularly advantageous. It is indeed well known that most cancers, if detected sufficiently early, may be treated with a high chance of recovery.

According to a further embodiment of the present invention, the disease which is to be diagnosed is an infectious disease. Indeed, several important functions during an infectious challenge (e.g. phagocytosis, intracellular killing of ingested micro organisms, and immunoregulatory activities) are performed by cells of the mononuclear phagocytes system, generating a pre-inflammatory phase. Examples of infectious diseases with a pre-inflammatory phase are otitis due to Gram negative bacilli such as Pseudomonas aeruginosa, Proteus mirabilis, Staphylococcus species, Streptococcus species and other. Viral infections are another example.

According to the present invention, the methods may also be carried out in order to diagnose a risk of cystic fibrosis, to diagnose the pre-inflammatory phase of cystic fibrosis.

For diagnosis of cystic fibrosis, a particularly preferred marker to be used in the methods of the invention is Ela1 or its mammalian orthologs, especially its human ortholog elastase 1 (ELA1).

The methods of the invention allow to diagnose a disease or a predisposition to a disease having a pre-inflammatory phase very early, before any clinical symptom of said disease. For example, in the case of type 1 diabetes, the diagnostic methods of the invention may be performed as early as on an infant of 2 years old, or even before. The diagnosis is thus achieved before the disease becomes symptomatic, preferably at the pre-inflammatory stage before any injury generated by the disease.

The marker used in the diagnostic methods of the invention is preferably a protein chosen in the group consisting of the following murine proteins: Pap, Reg3a, Reg2, Cel, Reg1, Tff2, Clps, Spp1, Klk9, Klk6, Rib1, Klk5, Muc1, Cckar, Ggh, Ang, Nucb2, Pnliprp2, Pla2g1b, Ela1, Ela2, 2210010C04Rik, Pnliprp1, Itmap1, Vtn, C1qb, Sycn, Amy1, Ctrb1, 1110002023Rik, 1810014L12Rik and their respective mammalian orthologs.

According to an aspect of the present invention, the marker is to be chosen amongst the following murine proteins Pap, Reg3a, Reg2, Cel, Reg1, Tff2, Clps and Spp1, and their respective orthologs in other mammals. The chosen marker is thus a protein implicated in tissue regeneration or integrity.

According to a different aspect of the invention, the marker is to be chosen amongst the following murine proteins Klk9, Klk6, Rib1, Klk5, Muc1, Cckar, Ggh, Ang, Nucb2 and Spp1 and their respective orthologs in other mammals. The chosen marker is thus a protein implicated in tumorigenesis.

According to yet another aspect of the invention, the marker is to be chosen amongst the following murine proteins: Pnliprp2, Pla2g1b, Ela1, Ela2, 2210010C04Rik, Pnliprp1, ltmap1, Vtn, C1qb and Spp1, and their respective homologs in other mammals. The chosen marker is thus a protein having an immune function.

According to yet another aspect of the invention, the marker is to be chosen amongst the following murine proteins: Sycn, Amy1, Ctrb1, 1110002023Rik and 1810014L12Rik, and their respective homologs in other mammals.

A particularly preferred marker according to the present invention is the murine protein elastase 1 and its respective homologs in other species of mammal. Elastase 1 is indeed expressed in neutrophils and neutrophils have been implicated in the development of vascular diseases. Moreover, according to the literature, this protein has been shown regulated in different pathologies, comprising cancer, infection and autoimmunity.

With regard to the body fluid which is used in the methods of the invention, said fluid may be *inter alia* blood, serum, plasma, urine, saliva, sweat or synovial fluid. According to a preferred embodiment of the invention, the fluid in which the concentration or level of the marker is to be determined is peripheral blood.

Alternatively, it is also envisaged in the context of the present invention, that the marker used in the methods is the transcript of the gene coding for one of the murine proteins previously mentioned or for a mammalian ortholog. The level of such a marker is preferably determined in a sample of a tissue, for example obtained after a biopsy, e.g. taken from the pancreatic lymph node of the mammal to be diagnosed. This embodiment is particularly suited for the diagnosis of a cancer having a pre-inflammatory phase. The tissue or organ in which the level of the marker is to be determined is preferably the tissue or organ suspected to contain tumour cells.

According to a particularly preferred embodiment of the present invention, the mammal which is to be diagnosed is a human being. This patient may be a young person, preferably a child, most preferably a young or very young child, especially a child less than five or less than two years old. The patient may alternatively be an adult.

The diagnostic methods are preferably repeated regularly, for example every 6 months, especially for patients initially negatively diagnosed but coming from a family comprising diseased members. When the patient to be diagnosed is a human, the marker is to be chosen amongst the human orthologs of the murine proteins previously mentioned. The marker is thus to be chosen in the group comprising the following human proteins: Regenerating islet-derived 3 alpha (REG3A), Pancreatitis associated protein 1 (PAP), Regenerating protein I beta, prostrate Kallikrein 2 (KLK2), Syncollin (SYCN), Pancreatic lipase-related protein 1 (PNLIPRP1), Kallikrein6 (KLK6), pancreatic Ribonuclease, RNase A family, 1 (RNASE1), Spasmolytic polypeptide (SP), Cholecystokinin A receptor, CUB and zona pellucida-like domains 1 (CUZD1 or ERG-1), pancreatic Colipase, (CLPS), pancreatic lipase-related protein 2, Lithostathine (REG1A), Amylase 1, Osteopontin (OPN), Phospholipase A2, group IB (PLA2G1B), Carboxyl ester lipase (bile salt-stimulated lipase) (CEL), Elastase 1 or Elastase 1 precursor (ELA1), Chymotrypsinogen B1 (CTRB1), Pancreatic elastase IIA, Protease, serine, 3 (mesotrypsin) (PRSS3), transmembrane Mucin 1 (MUC1), gamma-glutamyl hydrolase (conjugase, folylpolygammaglutamyl hydrolase) (GGH), Angiogenin, FK506 binding protein 11 (FKBP11), NT2RP2, Complement component 1, q subcomponent, beta polypeptide (C1QB), Nucleobindin 2 (NUCB2) and Vitronectin (serum spreading factor, somatomedin B,complement S-protein) (VTN). Example 2 details the human orthologs of the murine proteins previously mentioned, as well as their chromosomal location.

In one aspect of the method, the marker protein is preferably chosen in the group comprising: Regenerating islet-derived 3 alpha (REG3A), Pancreatitis associated protein 1 (PAP), Regenerating protein I beta, Spasmolytic polypeptide (SP), pancreatic Colipase, (CLPS), Lithostathine (REG1A), Carboxyl ester lipase (bile salt-stimulated lipase) (CEL) and Osteopontin. This marker protein is advantageously a protein known for its implication in tissue regeneration or integrity.

In another aspect, the marker protein is preferably chosen in the group comprising prostrate Kallikrein 2 (KLK2), Kallikrein6 (KLK6), pancreatic Ribonuclease, RNase A family, 1 (RNASE1), Cholecystokinin A receptor, transmembrane Mucin 1 (MUC1), gamma-glutamyl hydrolase (conjugase, folylpolygammaglutamyl hydrolase) (GGH), Angiogenin, Nucleobindin 2 (NUCB2) and Osteopontin. This marker protein is advantageously a protein known for its implication in tumorigenesis.

According to another aspect of the invention, the marker protein is preferably chosen in the group comprising Pancreatic lipase-related protein 1 (PNLIPRP1), CUB and zona pellucida-like domains 1 (CUZD1 or ERG-1), pancreatic lipase-related protein 2, Phospholipase A2, group IB (PLA2G1B), Elastase 1 or Elastase 1 precursor (ELA1), Pancreatic elastase IIA, Protease, serine, 3 (mesotrypsin) (PRSS3), Complement component 1, q subcomponent, beta polypeptide (C1QB), Vitronectin (serum spreading factor, somatomedin B,complement S-protein) (VTN) and Osteopontin. This marker protein is advantageously a protein known for having an immune function.

According to still another aspect of the invention, the marker protein is preferably chosen in the group comprising Syncollin (SYCN), Amylase 1, Chymotrypsinogen B1 (CTRB1), FK506 binding protein 11 (FKBP11) and NT2RP2.

According to a preferred embodiment, the marker protein is Elastase 1 or Elastase 1 precursor (ELA1).

In another embodiment of the invention, the mammals likely to be diagnosed are farm animals or pets, for example cats, dogs, horses, cattle or mice. For a mammal of a given species which is to be diagnosed, the marker is to be chosen amongst the ortholgs, when orthologs exist, in said species, of the following murine proteins Pap, Reg3a, Reg2, Cel, Reg1, Tff2, Clps, Spp1, Klk9, Klk6, Rib1, Klk5, Muc1, Cckar, Ggh, Ang, Nucb2, Pnliprp2, Pla2g1b, Ela1, Ela2, 2210010C04Rik, Pnliprp1, Itmap1, Vtn, C1qb, Sycn, Amy1, Ctrb1, 1110002O23Rik, 1810014L12Rik, or amongst the orthologs, in said species, of the following human proteins: Regenerating islet-derived 3 alpha (REG3A), Pancreatitis associated protein 1 (PAP), Regenerating protein I beta, prostrate Kallikrein 2 (KLK2), Syncollin (SYCN), Pancreatic lipase-related protein 1 (PNLIPRP1), Kallikrein6 (KLK6), pancreatic Ribonuclease, RNase A family, 1 (RNASE1), Spasmolytic polypeptide (SP), Cholecystokinin A receptor, CUB and zona pellucida-like domains 1 (CUZD1 or ERG-1), pancreatic Colipase, (CLPS), pancreatic lipase-related protein 2, Lithostathine (REG1A), Amylase 1, Osteopontin, Phospholipase A2, group IB (PLA2G1 B), Carboxyl ester lipase (bile salt-stimulated lipase) (CEL), Elastase 1 or Elastase 1 precursor (ELA1), Chymotrypsinogen B1 (CTRB1), Pancreatic elastase IIA, Protease, serine, 3 (mesotrypsin) (PRSS3), transmembrane Mucin 1 (MUC1), gamma-glutamyl hydrolase (conjugase, folylpolygammaglutamyl hydrolase) (GGH), Angiogenin, FK506 binding protein 11 (FKBP11), NT2RP2, Complement component 1, q subcomponent, beta polypeptide (C1QB), Nucleobindin 2 (NUCB2) and Vitronectin (serum spreading factor, somatomedin B,complement S-protein) (VTN).

A method according to the present invention comprises the step of measuring the level of a marker protein and the comparison of the measured level to a reference level for the marker protein. A method of the invention may also advantageously comprise the measure of the level of more than one marker protein. Thus, according to another embodiment of the invention, the methods comprise the measure of the level of two different marker proteins and their comparison to their respective reference levels. Both markers are to be chosen in the group comprising the following murine proteins Pap, Reg3a, Reg2, Cel, Reg1, Tff2, Clps, Spp1, Klk9, Klk6, Rib1, Klk5, Muc1, Cckar, Ggh, Ang, Nucb2, Pnliprp2, Pla2g1b, Ela1, Ela2, 2210010C04Rik, Pnliprp1, Itmap1, Vtn, C1qb, Sycn, Amy1, Ctrb1, 1110002O23Rik, 1810014L12Rik, and the orthologs of said proteins in other mammalian species. Both measures may be done simultaneously, for example by an automat, or sequentially, the comparison steps may be carried out after both measures, or alternatively, the measure of the level of a first marker is followed by the comparison to the reference level before the measure step for the second marker is carried out. According to this embodiment, steps a) and b) of the methods of the invention are repeated twice, for two different markers. It may also be envisaged that the steps are repeated twice with the same marker, in order to minimise the possibility of false diagnosis. In this case, the measures are advantageously carried out on two different samples.

When steps a) and b) of the methods of the invention are repeated twice, the later onset of said disease, or disease-prone state, is preferably diagnosed only if both measured levels are significantly divergent from the respective reference levels. It may also be envisaged that the later onset of said disease, or disease-prone state, is diagnosed if only one measured level is divergent from its reference level. In this last case, the method may advantageously be repeated with a third marker, comprised in the markers recited above.

According to another embodiment of the invention, steps a) and b) of the methods are repeated three times, simultaneously or sequentially as mentioned above, with three markers. Said markers are to be chosen in the group comprising the following murine proteins Pap, Reg3a, Reg2, Cel, Reg1, Tff2, Clps, Spp1, Klk9, Klk6, Rib1, Klk5, Muc1, Cckar, Ggh, Ang, Nucb2, Pnliprp2, Pla2g1b, Ela1, Ela2, 2210010C04Rik, Pnliprp1, Itmap1, Vtn, C1qb, Sycn, Amy1, Ctrb1, 1110002O23Rik, 1810014L12Rik, and the orthologs of said proteins in other mammalian species. Preferably, said three marker proteins are distinct from each other. Moreover, it is also preferred that the later onset of said disease, or disease-prone state, is diagnosed only if all measured levels are significantly divergent from their respective reference levels, or at least 2 of 3.

The present invention also encompasses methods wherein steps a) and b) as defined above are repeated four times, five times or even more, for example 7 times or 10 times. The markers may be chosen in the same group as defined above, they are preferably distinct from each other. The later onset of said disease, or disease-prone state, is preferably diagnosed only if all measured levels are significantly divergent from their respective reference levels, or if a majority of the levels are significantly divergent from their respective reference levels, or at least two levels are significantly divergent from their respective reference levels.

With regard to the association of markers, which are to be measured together according to this embodiment of the invention, this association comprises at least two different markers, preferably at least 3, 4 or 5, for example 5, 7 or 10. Such an association comprises preferably at least two markers in at least two differents groups, the groups being defined as followed:
Group 1: the following murine proteins Pap, Reg3a, Reg2, Cel, Reg1, Tff2, Clps and Spp1, and their respective orthologs in other mammals,
Group 2: the following murine proteins Klk9, Klk6, Rib1, Klk5, Muc1, Cckar, Ggh, Ang, Nucb2 and Spp1 and their respective orthologs in other mammals,
Group 3: the following murine proteins: Pnliprp2, Pla2g1b, Ela1, Ela2, 2210010C04Rik, Pnliprp1, Itmap1, Vtn, C1qb and Spp1, and their respective homologs in other mammals,
Group 4: the following murine proteins: Sycn, Amy1, Ctrb1, 1110002O23Rik and 1810014L12Rik, and their respective homologs in other mammals.

An association of markers may comprise at least a marker in each of the four groups, or at least a marker in each of the first 3 groups.

Alternatively, an association of markers may comprise proteins encoded by genes located at the same locus on the chromosome. The localization of the genes corresponding to the murine and human markers of the invention is detailed in example 2.

For human diagnosis, it may be advantageous to make an association of different markers positioned on human chromosome 9, or on human chromosome 10, or on human chromosome 2 or on human chromosome 19.

Alternatively, the association of markers may associate marker proteins having similar function, for example Reg and Pap, or the kallikrein proteins, or the lipase proteins.

According to a preferred embodiment, an association of markers comprises at least spp1, or its human ortholog Osteopontin.

Another preferred association of marker proteins comprises or consists in Spp1 and Ela 1, for example Spp1 with Ela1 and Ela 2 and Sycn, or their respective human orthologs.

With regard to the measure step a), any method well known from the skilled person in the domain can be used. Should the marker be an RNA, different methods can be used, as for example the use of chip with RNA probes.

Should the marker be a protein, use can advantageously be made of a labelled ligand, said ligand specifically binding the chosen protein. Such a ligand is for example a natural ligand, for example a target of the protein. A particularly preferred type of ligand is antibody. Labelled antibodies specifically directed against the chosen marker protein are indeed suited for determining the level of said marker protein in a sample of a body fluid. A preferred antibody is a monoclonal antibody, although polyclonal antibodies may also be used.

When an antibody against a marker protein is used in order to determine the level of said marker protein in a sample, said antibody is not necessarily labelled. If said antibody is for example from another species than the mammal which is diagnosed, the antibody may be detected by a further antibody.

Fluorescent labels are particularly preferred in the context of the present invention for the ligand allowing the detection of the chosen marker protein, and the determination of its level.

A method which can be used for measuring the level of a marker protein of the invention is the so-called ELISA (Enzyme Linked ImmunoSorbent Assay), as exemplified in Koopmann et al (2004).

When the mammal to be diagnosed is a human being, the ligand is preferably chosen amongst the antibodies mentioned in example 3. These antibodies are commercially available antibodies, which can be identified and obtained without difficulty by a skilled person in the domain of the present invention.

The ligand may be chosen *inter alia* amongst the following list of antibodies: anti-pap antibody (Rec Human REG1A: Cat No. RP-10087-P1ABX), anti-kallikrein 9 antibody (KLK9 (C-15): sc-20385 Santa Cruz), anti-kallikrein-6 antibody (KLK6 (N-16): sc-20376 Santa Cruz), anti-Trefoil factor 2 spasmolytic protein1 antibody (SP (P-19): sc-23558 Santa Cruz), anti-Cholecystokinin receptor antibody (CCK-AR (G-17): sc-16173 Santa Cruz), anti-kallikrein 5 antibody (KLK5 (N-14): sc-20375 Santa Cruz), anti-Islet Regenerating antibody (Rec Human REG1A: Cat No. RP-10087-P1ABX), anti-amylase 1 antibody (Amylase (G-10): sc-46657), anti-secreted phosphoprotein 1 antibody (Rabit polyclonal RB-9097) and anti-mucin 1 antibody (CD24 (GPI-linked surface mucin) Ab-1).

Moreover, antibodies against the marker proteins of the invention can also easily been obtained by well-known methods, either polyclonal or monoclonal antibodies. Technologies for obtaining humanized antibodies are also routine work for the skilled person in this domain.

The present invention also encompasses the use of a protein chosen amongst the following murine proteins Pap, Reg3a, Reg2, Cel, Reg1, Tff2, Clps, Spp1, Klk9, Klk6, Rib1, Klk5, Muc1, Cckar, Ggh, Ang, Nucb2, Pnliprp2, Pla2g1b, Ela1, Ela2, 2210010C04Rik, Pnliprp1, Itmap1, Vtn, C1qb, Sycn, Amy1, Ctrb1, 1110002023Rik and 1810014L12Rik, and their respective orthologs in different mammalian species, as a seric marker for the predisposition of a mammal to a disease having a pre-inflammatory phase. According to the invention, this seric marker is used to define a predisposition to the disease before any clinical signs can be detected.

The invention also concerns the use of a protein chosen amongst the following murine proteins Pap, Reg3a, Reg2, Cel, Reg1, Tff2, Clps, Spp1, Klk9, Klk6, Rib1, Klk5, Muc1, Cckar, Ggh, Ang, Nucb2, Pnliprp2, Pla2g1b, Ela1, Ela2, 2210010C04Rik, Pnliprp1, Itmap1, Vtn, C1qb, Sycn, Amy1, Ctrb1, 1110002O23Rik and 1810014L12Rik, and their respective orthologs in different mammalian species, as a seric marker for the early pre-inflammatory condition of a disease, especially in autoimmune diseases. According to the invention, this seric marker is used for detecting the early pre-inflammatory phase of an autoimmune disease, before any clinical signs in the mammal.

Particularly preferred seric markers for the uses of the invention are chosen amongst the following murine proteins: Pap, Reg3a, Reg2, Cel, Reg1, Tff2, Clps, Spp1, Klk9, Klk6, Rib1, Klk5, Muc1, Cckar, Pnliprp2, Pla2g1b, Ela1, Ela2, 2210010C04Rik, Pnliprp1, Itmap1, Sycn, Amy1 and Ctrb1, and their respective mammalian orthologs.

In one aspect of the uses of the invention, the protein is chosen amongst the following murine proteins Pap, Reg3a, Reg2, Cel, Reg1, Tff2, Clps and Spp1, and their respective orthologs in other mammals. The chosen protein used as a seric marker is advantageously implicated in tissue regeneration or integrity.

According to another aspect of the uses of the invention, the protein is chosen amongst the following murine proteins Klk9, Klk6, Rib1, Klk5, Muc1, Cckar, Ggh, Ang, Nucb2 and Spp1 and their respective orthologs in other mammalian species. The chosen protein used as a seric marker is advantageously implicated in tumorigenesis.

According to another aspect of the uses of the invention, the protein is chosen amongst the following murine proteins Pnliprp2, Pla2g1b, Ela1, Ela2, 2210010C04Rik, Pnliprp1, Itmap1, Vtn, C1qb and Spp1 and their respective orthologs in other mammalian species. The chosen protein used as a seric marker has advantageously an immune function.

According to still another aspect of the uses of the invention, the protein is chosen amongst the following murine proteins Sycn, Amy1, Ctrb1, 1110002023Rik and 1810014L12Rik and their respective orthologs in other mammals.

A preferred protein for use as a seric marker according to this invention is Elastase 1 and its orthologs in the other mammal species, especially human Elastase 1.

With regard to the disease having a pre-inflammatory phase, preferred diseases have already been disclosed above for the diagnostic methods of the invention. The seric marker of the invention is likely to be used in the detection of the pre-inflammatory stage of the same diseases. Autoimmune diseases and cancers are particularly preferred, especially in human. Type 1 diabetes is the mostly preferred autoimmune disease in human.

Indeed, the seric markers of the invention are particularly suited for the detection of a predisposition to a pathology having a pre-inflammatory phase in human beings. The seric marker is thus to be chosen amongst the human orthologs of the murine proteins disclosed above. The seric marker is thus to be chosen from the group comprising: Regenerating islet-derived 3 alpha (REG3A), Pancreatitis associated protein 1 (PAP), Regenerating protein I beta, prostrate Kallikrein 2 (KLK2), Syncollin (SYCN), Pancreatic lipase-related protein 1 (PNLIPRP1), Kallikrein6 (KLK6), pancreatic Ribonuclease, RNase A family, 1 (RNASE1), Spasmolytic polypeptide (SP), Cholecystokinin A receptor, CUB and zona pellucida-like domains 1 (CUZD1 or ERG-1), pancreatic Colipase, (CLPS), pancreatic lipase-related protein 2, Lithostathine (REG1A), Amylase 1, Osteopontin (OPN), Phospholipase A2, group IB (PLA2G1B), Carboxyl ester lipase (bile salt-stimulated lipase) (CEL), Elastase 1 or Elastase 1 precursor (ELA1), Chymotrypsinogen B1 (CTRB1), Pancreatic elastase IIA, Protease, serine, 3 (mesotrypsin) (PRSS3), transmembrane Mucin 1 (MUC1), gamma-glutamyl hydrolase (conjugase, folylpolygammaglutamyl hydrolase) (GGH), Angiogenin, FK506 binding protein 11 (FKBP11), NT2RP2, Complement component 1, q subcomponent, beta polypeptide (C1QB), Nucleobindin 2 (NUCB2) and Vitronectin (serum spreading factor, somatomedin B, complement S-protein) (VTN).

In one aspect of the uses of the invention, the protein is chosen amongst the following human proteins: Regenerating islet-derived 3 alpha (REG3A), Pancreatitis associated protein 1 (PAP), Regenerating protein I beta, Spasmolytic polypeptide (SP), pancreatic Colipase, (CLPS), Lithostathine (REG1A), Carboxyl ester lipase (bile salt-stimulated lipase) (CEL) and Osteopontin. This chosen protein used as a seric marker is advantageously implicated in tissue regeneration or integrity.

According to another aspect of the uses of the invention, the protein is chosen amongst the following human proteins: prostrate Kallikrein 2 (KLK2), Kallikrein6 (KLK6), pancreatic Ribonuclease, RNase A family, 1 (RNASE1), Cholecystokinin A receptor, transmembrane Mucin 1 (MUC1), gamma-glutamyl hydrolase (conjugase, folylpolygammaglutamyl hydrolase) (GGH), Angiogenin, Nucleobindin 2 (NUCB2) and Osteopontin. This chosen protein used as a seric marker is advantageously implicated in tumorigenesis.

According to another aspect of the uses of the invention, the protein is chosen amongst the following human proteins: Pancreatic lipase-related protein 1 (PNLIPRP1), CUB and zona pellucida-like domains 1 (CUZD1 or ERG-1), pancreatic lipase-related protein 2, Phospholipase A2, group IB (PLA2G1B), Elastase 1 or Elastase 1 precursor (ELA1), Pancreatic elastase IIA, Protease, serine, 3 (mesotrypsin) (PRSS3), Complement component 1, q subcomponent, beta polypeptide (C1QB), Vitronectin (serum spreading factor, somatomedin B,complement S-protein) (VTN) and Osteopontin. This chosen protein used as a seric marker has advantageously an immune function.

According to still another aspect of the uses of the invention, the protein is chosen amongst the following human proteins: Syncollin (SYCN), Amylase 1, Chymotrypsinogen B1 (CTRB1), FK506 binding protein 11 (FKBP11) and NT2RP2.

A marker protein is preferably used as a diagnostic marker of type 1 diabetes in human, especially for children as young as 5 years, preferably even younger, for example 18 or 24 months.

All the preferred embodiments of the methods of the invention are also appropriate for the uses of the invention as described above.

The invention also relates to the use of a ligand, which specifically binds a protein chosen from the group comprising the following murine proteins Pap, Reg3a, Reg2, Cel, Reg1, Tff2, Clps, Spp1, Klk9, Klk6, Rib1, Klk5, Muc1, Cckar, Ggh, Ang, Nucb2, Pnliprp2, Pla2g1b, Ela1, Ela2, 2210010C04Rik, Pnliprp1, Itmap1, Vtn, C1qb, Sycn, Amy1, Ctrb1, 1110002O23Rik and 1810014L12Rik, and their respective orthologs in different mammalian species, in a method for diagnosing, before any clinical signs, a predisposition to a disease having a pre-inflammatory phase in a mammal, or in a method for diagnosing said disease before any clinical signs.

The preferred mammals and preferred diseases according to this invention have been previously disclosed. The ligand used is preferably an antibody, particularly a monoclonal antibody. Said ligand is advantageously labelled, for example by a fluorescent label.

As a preferred embodiment of the invention concerns human diagnosis, the invention thus also relates to the use of a ligand, which specifically binds a protein chosen from the group consisting of: Regenerating islet-derived 3 alpha (REG3A), Pancreatitis associated protein 1 (PAP), Regenerating protein I beta, prostrate Kallikrein 2 (KLK2), Syncollin (SYCN), Pancreatic lipase-related protein 1 (PNLIPRP1), Kallikrein6 (KLK6), pancreatic Ribonuclease, RNase A family, 1 (RNASE1), Spasmolytic polypeptide (SP), Cholecystokinin A receptor, CUB and zona pellucida-like domains 1 (CUZD1 or ERG-1), pancreatic Colipase, (CLPS), pancreatic lipase-related protein 2, Lithostathine (REG1A), Amylase 1, Osteopontin (OPN), Phospholipase A2, group IB (PLA2G1B), Carboxyl ester lipase (bile salt-stimulated lipase) (CEL), Elastase 1 (or Elastase 1 precursor) (ELA1), Chymotrypsinogen B1 (CTRB1), Pancreatic elastase IIA, Protease, serine, 3 (mesotrypsin) (PRSS3), transmembrane Mucin 1 (MUC1), gamma-glutamyl hydrolase (conjugase, folylpolygammaglutamyl hydrolase) (GGH), Angiogenin, FK506 binding protein 11 (FKBP11), NT2RP2, Complement component 1, q subcomponent, beta polypeptide (C1QB), Nucleobindin 2 (NUCB2) and Vitronectin (serum spreading factor, somatomedin B, complement S-protein) (VTN) in a method for diagnosing, before any clinical signs, a predisposition to a disease having a pre-inflammatory phase in a human patient, or in a method for diagnosing said disease before any clinical signs.

The diseases having a pre-inflammatory phase have been recited in the previous aspects of the invention, they comprise autoimmune diseases, cancers and infection. Autoimmune diseases are particularly preferred in the context of the present invention, especially type 1 diabetes mellitus, multiple sclerosis, rheumatoid arthritis, collagen induced arthritis and autoimmune hepatitis.

A preferred class of patients, human or other mammals, likely to be diagnosed using a ligand as described, is the class of patients presenting insulin autoantibodies in their peripheral blood.

A preferred ligand for use in a method of diagnosing is a ligand specifically binding Elastase 1.

The present invention also encompasses therapeutic applications using at least one of the following murine proteins Pap, Reg3a, Reg2, Cel, Reg1, Tff2, Clps, Spp1, Klk9, Klk6, Rib1, Klk5, Muc1, Cckar, Ggh, Ang, Nucb2, Pnliprp2, PIa2g1b, Ela1, Ela2, 2210010C04Rik, Pnliprp1, Itmap1, Vtn, C1qb, Sycn, Amy1, Ctrb1, 1110002023Rik and 1810014L12Rik, or their respective orthologs in different mammalian species, as a therapeutic target. Said therapeutic applications are preferably for human therapy, the target protein is thus to be chosen in the human orthologs of the murine proteins recited above.

Preferred embodiments are a protein chosen from Regenerating islet-derived 3 alpha (REG3A), Pancreatitis associated protein 1 (PAP), Regenerating protein I beta, Syncollin (SYCN), Pancreatic lipase-related protein 1 (PNLIPRP1), pancreatic Ribonuclease, RNase A family, 1 (RNASE1), Spasmolytic polypeptide (SP), Cholecystokinin A receptor, CUB and zona pellucida-like domains 1 (CUZD1 or ERG-1), pancreatic Colipase, (CLPS), pancreatic lipase-related protein 2, Lithostathine (REG1A), Amylase 1, Osteopontin, Phospholipase A2, group IB (PLA2G1B), Carboxyl ester lipase (bile salt-stimulated lipase) (CEL), Elastase 1 (or Elastase 1 precursor) (ELA1), Chymotrypsinogen B1 (CTRB1), Pancreatic elastase IIA, Protease, serine, 3 (mesotrypsin) (PRSS3), transmembrane Mucin 1 (MUC1), gamma-glutamyl hydrolase (conjugase, folylpolygammaglutamyl hydrolase) (GGH), Angiogenin, FK506 binding protein 11 (FKBP11), NT2RP2, Complement component 1, q subcomponent, beta polypeptide (C1QB), Nucleobindin 2 (NUCB2) and Vitronectin (serum spreading factor, somatomedin B complement S-protein) (VTN) for use in therapy.

The present invention concerns an agent capable of modulating the activity of a protein chosen from the group comprising: Regenerating islet-derived 3 alpha (REG3A), Pancreatitis associated protein 1 (PAP), Regenerating protein I beta, prostrate Kallikrein 2 (KLK2), Syncollin (SYCN), Pancreatic lipase-related protein 1 (PNUPRP1), Kallikrein6 (KLK6), pancreatic Ribonuclease, RNase A family, 1 (RNASE1), Spasmolytic polypeptide (SP), Cholecystokinin A receptor, CUB and zona pellucida-like domains 1 (CUZD1 or ERG-1), pancreatic Colipase, (CLPS), pancreatic lipase-related protein 2, Lithostathine (REG1A), Amylase 1, Osteopontin, Phospholipase A2, group IB (PLA2G1B), Carboxyl ester lipase (bile salt-stimulated lipase) (CEL), Elastase 1 (or Elastase 1 precursor) (ELA1), Chymotrypsinogen B1 (CTRB1), Pancreatic elastase IIA, Protease, serine, 3 (mesotrypsin) (PRSS3), transmembrane Mucin 1 (MUC1), gamma-glutamyl hydrolase (conjugase, folylpolygammaglutamyl hydrolase) (GGH), Angiogenin, FK506 binding protein 11 (FKBP11), NT2RP2, Complement component 1, q subcomponent, beta polypeptide (C1QB), Nucleobindin 2 (NUCB2) and Vitronectin (serum spreading factor, somatomedin B complement S-protein) (VTN), for use in therapy, especially for use in treating patients suffering from a disease having a pre-inflammatory phase, preferably an autoimmune disease, and most preferably suffering from type 1 diabetes. The invention also encompasses such an agent for a prophylactic use for disease-prone patients.

The invention also concerns the use of an agent capable of modulating the activity of a protein chosen from the group consisting of: Regenerating islet-derived 3 alpha (REG3A), Pancreatitis associated protein 1 (PAP), Regenerating protein I beta, prostrate Kallikrein 2 (KLK2), Syncollin (SYCN), Pancreatic lipase-related protein 1 (PNUPRP1), Kallikrein6 (KLK6), pancreatic Ribonuclease, RNase A family, 1 (RNASE1), Spasmolytic polypeptide (SP), Cholecystokinin A receptor, CUB and zona pellucida-like domains 1 (CUZD1 or ERG-1), pancreatic Colipase, (CLPS), pancreatic lipase-related protein 2, Lithostathine (REG1A), Amylase 1, Osteopontin, Phospholipase A2, group IB (PLA2G1B), Carboxyl ester lipase (bile salt-stimulated lipase) (CEL), Elastase 1 (ELA1), Chymotrypsinogen B1 (CTRB1), Pancreatic elastase IIA, Protease, serine, 3 (mesotrypsin) (PRSS3), transmembrane Mucin 1 (MUC1), gamma-glutamyl hydrolase (conjugase, folylpolygammaglutamyl hydrolase) (GGH), Angiogenin, FK506 binding protein 11 (FKBP11), NT2RP2, Complement component 1, q subcomponent, beta polypeptide (C1QB), Nucleobindin 2 (NUCB2) and Vitronectin (serum spreading factor, somatomedin B complement S-protein) (VTN), for the manufacture of a medicament for treating patients suffering from a disease having a pre-inflammatory phase, preferably an autoimmune disease, and most preferably suffering from type 1 diabetes.

The invention also concerns the use of an agent capable of modulating the activity of a protein chosen amongst the following human proteins: Regenerating islet-derived 3 alpha (REG3A), Pancreatitis associated protein 1 (PAP), Regenerating protein I beta, prostrate kallikrein 2 (KLK2), Syncollin (SYCN), Pancreatic lipase-related protein 1 (PNLIPRP1), Kallikrein6 (KLK6), pancreatic Ribonuclease, RNase A family, 1 (RNASE1), Spasmolytic polypeptide (SP), Cholecystokinin A receptor, CUB and zona pellucida-like domains 1 (CUZD1 or ERG-1), pancreatic Colipase, (CLPS), pancreatic lipase-related protein 2, Lithostathine (REG1A), Amylase 1, Osteopontin, Phospholipase A2, group IB (PLA2G1B), Carboxyl ester lipase (bile salt-stimulated lipase) (CEL), Elastase 1 (ELA1), Chymotrypsinogen B1 (CTRB1), Pancreatic elastase IIA, Protease, serine, 3 (mesotrypsin) (PRSS3), transmembrane Mucin 1 (MUC1), gamma-glutamyl hydrolase (conjugase, folylpolygammaglutamyl hydrolase) (GGH), Angiogenin, FK506 binding protein 11 (FKBP11), NT2RP2, Complement component 1, q subcomponent, beta polypeptide (C1QB), Nucleobindin 2 (NUCB2) and Vitronectin (serum spreading factor, somatomedin B,complement S-protein) (VTN), for the manufacture of a medicament for treating patients, being disease-prone for a disease having a pre-inflammatory phase, preferably for an autoimmune disease, and most preferably disease-prone for type 1 diabetes, before clinical signs.

According to an aspect of these therapeutic applications of the invention, the protein is chosen from the group consisting of the following human proteins: Regenerating islet-derived 3 alpha (REG3A), Pancreatitis associated protein 1 (PAP), Regenerating protein I beta, Spasmolytic polypeptide (SP), pancreatic Colipase, (CLPS), Lithostathine (REG1A), Carboxyl ester lipase (bile salt-stimulated lipase) (CEL) and Osteopontin. The chosen protein is advantageously known for its implication in tissue regeneration or integrity.

According to another aspect of these therapeutic applications, the protein is chosen from the group consisting of the following human proteins: prostrate Kallikrein 2 (KLK2), Kallikrein6 (KLK6), pancreatic Ribonuclease, RNase A family, 1 (RNASE1), Cholecystokinin A receptor, transmembrane Mucin 1 (MUC1), gamma-glutamyl hydrolase (conjugase, folylpolygammaglutamyl hydrolase) (GGH), Angiogenin, Nucleobindin 2 (NUCB2) and

Osteopontin, and most preferably chosen amongst the group consisting of pancreatic Ribonuclease, RNase A family, 1 (RNASE1), Cholecystokinin A receptor, transmembrane Mucin 1 (MUC1), gamma-glutamyl hydrolase (conjugase, folylpolygammaglutamyl hydrolase) (GGH), Angiogenin, Nucleobindin 2 (NUCB2) and Osteopontin. The chosen protein is advantageously known for its implication in tumorigenesis.

According to a further aspect of these therapeutic applications, the protein is chosen from the group consisting of the following human proteins: Pancreatic lipase-related protein 1 (PNLIPRP1), CUB and zona pellucida-like domains 1 (CUZD1 or ERG-1), pancreatic lipase-related protein 2, Phospholipase A2, group IB (PLA2G1B), Elastase 1 (or Elastase 1 precursor) (ELA1), Pancreatic elastase IIA, Protease, serine, 3 (mesotrypsin) (PRSS3), Complement component 1, q subcomponent, beta polypeptide (C1QB), Vitronectin (serum spreading factor, somatomedin B,complement S-protein) (VTN) and Osteopontin. The protein is advantageously known as having an immune function.

According to yet another aspect of the therapeutic applications of the present invention, the protein is chosen from the group of Syncollin (SYCN), Amylase 1, Chymotrypsinogen B1 (CTRB1), FK506 binding protein 11 (FKBP11) and NT2RP2.

A particularly preferred protein is the human Elastase 1.

In the context of the present invention, the modulation induced by the agent is an inhibition of the protein activity or an enhancement of said activity, but preferably inhibition. This agent may modulate the activity of the chosen protein either by direct or indirect interaction with the protein, or by interaction with the DNA or RNA transcript of the protein. A suitable agent capable of inhibiting the protein activity is for example a ligand which is a competitor of the natural ligands of the protein. Such an agent is for example an antibody, preferably a monoclonal antibody. Alternatively, the agent may intervene at the translation level by inhibiting the translation of the RNA. Such an agent is for example a ribozyme or an RNAi. According to another aspect, the agent may also intervene at the DNA level, for example by inhibiting the initiation of the transcription.

A preferred agent is an antibody, especially a monoclonal antibody. A suitable agent which can be used in the therapeutic applications of the present invention, is preferably an antibody chosen amongst the commercially availabe antibodies directed against the above-mentioned human proteins. Some of these antibodies are exemplified in the experimental section (example 3). An agent can be chosen for example from the following list of antibodies: Antibody anti-pap (Rec Human REG1A: Cat No. RP-10087-P1ABX), antibody anti-kallikrein 9 (KLK9 (C-15): sc-20385 Santa Cruz), antibody anti-kallikrein-6 (KLK6 (N-16): sc-20376 Santa Cruz), antibody anti-Trefoil factor 2 spasmolytic protein1 (SP (P-19): sc-23558 Santa Cruz), antibody anti-Cholecystokinin receptor (CCK-AR (G-17): sc-16173 Santa Cruz), antibody anti-kallikrein 5 (KLK5 (N-14): sc-20375 Santa Cruz), antibody anti-Islet Regenerating (Rec Human REG1A: Cat No. RP-10087-P1ABX), antibody anti-amylase 1 (Amylase (G-10): sc-46657), antibody anti-secreted phosphoprotein 1 (Rabit polyclonal RB-9097) and antibody anti-mucin 1 (CD24 (GPI-linked surface mucin) Ab-1).

Moreover, obtaining antibodies against one of these proteins is routine work for a skilled person.

According to another aspect, the agent may enhance the activity of said protein. The enhancement may also be an enhancement at the DNA level, at the translation level or at the protein level. An agent capable of enhancing the activity of a protein of the invention may be the same protein, the activity is thus enhanced by increasing the concentration of the protein.

According to a preferred embodiment of the therapeutic uses of the invention, the patient which is to be treated by an agent as described has not yet evident sign of insulitis or mild-insulitis, therefore the patient is still in the early stages of type 1 diabetes.

The patient which is to be treated according to the invention is preferably under 10 years, more preferably under 5 years, the patient may also be as young as 18 or 24 months.

The present invention also concerns a kit for diagnosing a predisposition to a disease having a pre-inflammatory phase. The kit is to be used for diagnosing such a disease in a patient before any clinical signs of the disease. A kit according to the invention comprises:
- a means for dosing a protein chosen from the group comprising: Regenerating islet-derived 3 alpha (REG3A), Pancreatitis associated protein 1 (PAP), Regenerating protein I beta, prostrate kallikrein 2 (KLK2), Syncollin (SYCN), Pancreatic lipase-related protein 1 (PNUPRP1), Kallikrein6 (KLK6), pancreatic Ribonuclease, RNase A family, 1 (RNASE1), Spasmolytic polypeptide (SP), Cholecystokinin A receptor, CUB and zona pellucida-like domains 1 (CUZD1 or ERG-1), pancreatic Colipase, (CLPS), pancreatic lipase-related protein 2, Lithostathine (REG1A), Amylase 1, Osteopontin, Phospholipase A2, group IB (PLA2G1B), Carboxyl ester lipase (bile salt-stimulated lipase) (CEL), Elastase 1 (ELA1) (or Elastase 1 precursor), Chymotrypsinogen B1 (CTRB1), Pancreatic elastase IIA, Protease, serine, 3 (mesotrypsin) (PRSS3), transmembrane Mucin 1 (MUC1), gamma-glutamyl hydrolase (conjugase, folylpolygammaglutamyl hydrolase) (GGH), Angiogenin, FK506 binding protein 11 (FKBP11), NT2RP2, Complement component 1, q subcomponent, beta polypeptide (C1QB), Nucleobindin 2 (NUCB2) and Vitronectin (serum spreading factor, somatomedin B, complement S-protein) (VTN), or a mammalian ortholg thereof and
- a reference level for said protein.

The reference level is as defined previously.

The preferred embodiments already mentioned for the various methods and uses of the invention are also applicable to the kit. Especially, a kit according to the invention is preferably used to diagnose a predisposition to an autoimmune disease, a cancer or an infection. Preferred autoimmune diseases are type 1 diabetes mellitus, multiple sclerosis, rheumatoid arthritis, collagen induced arthritis and autoimmune hepatitis.

The patient to be diagnosed by said kit is preferably a person from a family presenting high risk of potential autoimmune diseases or cancer. A patient is preferably a child, for example less than 10 years. The kit may advantageously be used for diagnosing child as young as 5 years or even less, for example for diagnosing children with 18 or 24 months. Indeed, for various autoimmune diseases like type 1 diabetes, the preclinical stage may begin early in the life of patients, as early as 2 years and even before. It is thus very important to be able, thanks to the kit of the invention, to diagnose the pathology at this preclinical stage, in order to avoid, delay or attenuate the further symptoms.

The means for dosing the protein chosen, in a kit of the invention, is preferably a ligand which specifically binds the chosen protein. Said ligand may advantageously be labelled, for example by a fluorescent label.

In the context of the present invention, preferred ligands are antibodies, especially monoclonal antibodies. An antibody comprised in a kit of the invention can be chosen *inter alia* from the antibodies detailed in example 3 of the present application. It can be chosen for example from the following list of antibodies: Antibody anti-pap (Rec Human REG1A: Cat No. RP-10087-P1ABX), antibody anti-kallikrein 9 (KLK9 (C-15): sc-20385 Santa Cruz), antibody anti-kallikrein-6 (KLK6 (N-16): sc-20376 Santa Cruz), antibody anti-Trefoil factor 2 spasmolytic protein1 (SP (P-19): sc-23558 Santa Cruz), antibody anti-Cholecystokinin receptor (CCK-AR (G-17): sc-16173 Santa Cruz), antibody anti-kallikrein 5 (KLK5 (N-14): sc-20375 Santa Cruz), antibody anti-Islet Regenerating (Rec Human REG1A: Cat No. RP-10087-P1ABX), antibody anti-amylase 1 (Amylase (G-10): sc-46657), antibody anti-secreted phosphoprotein 1 (Rabit polyclonal RB-9097) and antibody anti-mucin 1 (CD24 (GPI-linked surface mucin) Ab-1).

The reference level provided in the kit may be materialized by a line on the test tube of the kit or may be indicated in the instructions for users.

### Legends of Figures

**Figure 1**. Phenotypic windows as defined by IAA: **A**. Diabetic Risk as % of diabetic animals versus age of diabetes appearance. **B**. Temporal Evolution of disease as marked by the presence of Insulin Auto Antibodies at various ages starting from birth (time 0). **W-I**: Phenotypic Window I between 3 to 5 weeks; **W-II:** Phenotypic Window II between 8 to 12 weeks and **W-III:** Phenotytpic Window III after 10 weeks of age {Adapted from Melanitou, 2004}.
**Figure 2:** Data Analysis- Genespring GS6.0-II
   Normalization:
   - per chip normalization: normalize to the distribution of all genes using 50th percentile.
   - per gene normalization: normalize to the median for each gene (a cut-off value of 0,001).
   - make minimum value 0
   Filtering:
   - Control strength CS>60
   - Genes not changing (generated by using the drawn gene function in which this artificial gene was a horizontal line)
   - Genes present in at least 4 out of 9 samples
   - Plot data by Venn diagram.
   See materials and methods for additional information.

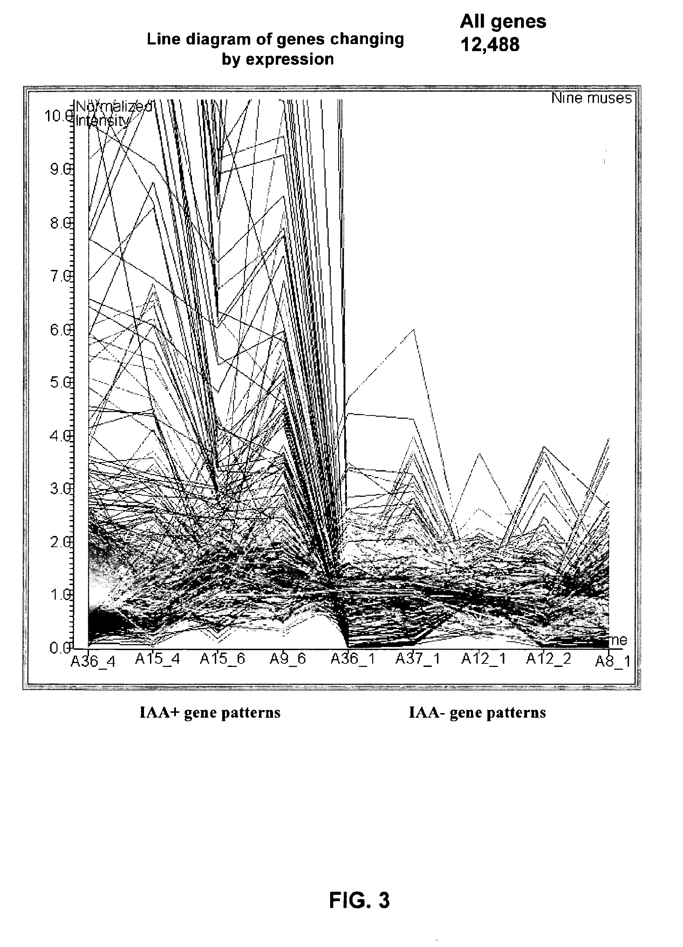

**Figure 3: Distribution of gene expression patterns** in PaLN of IAA+ and IAA- NOD mice. All genes spotted on the Affymetrix Arrays MU74A version 2 are taken in consideration. To note the points placed by the program according to their gene expression differences out of the median line. These genes correspond to the differentially expressed genes between the two sets of samples. To be noted also the smaller differences found in the differentially expressed genes in the IAA negative samples.

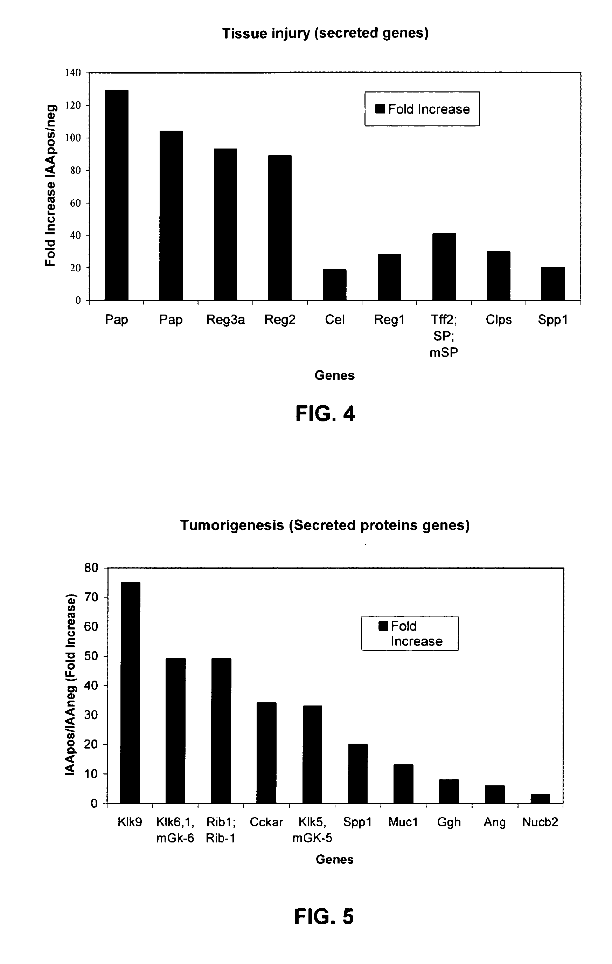

**Figure 4**: Fold increase of genes potentially implicated in **tissue integrity and regeneration** (Group I) and differentially expressed between IAA pos and IAA neg NOD mice in the PaLN.
**Figure 5:** Fold increase of genes potentially implicated **in tumorigenesis** (Group II) and found to be differentially expressed between IAA pos and IAA neg NOD mice in the PaLN.

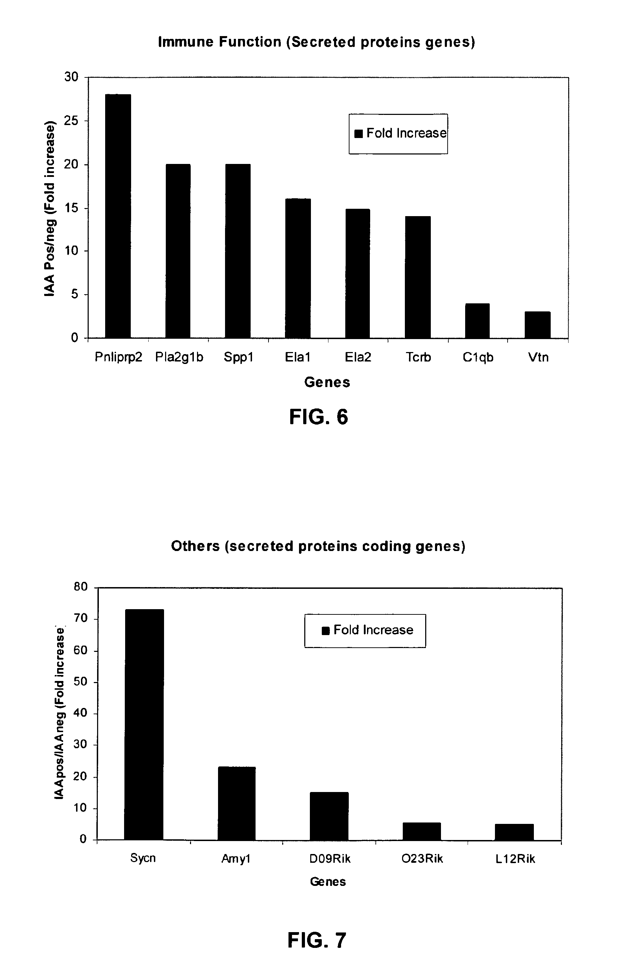

**Figure 6:** Fold increase of genes potentially implicated in **immune function** (Group III) and found to be differentially expressed between IAA pos and IAA neg NOD mice in the PaLN.
**Figure 7:** Fold increase of genes in the **unclassified** (Group IV) and found to be differentially expressed between IAA pos and IAA neg NOD mice in the PaLN.

### EXAMPLES:

### Example 1:

The reported genes have been identified by analysis of expression patterns between NOD (Non-Obese Diabetes) diabetes prone mice positive or negative for the presence of Insulin Auto Antibodies (IAA) at an early age (at 5 weeks). The aim of these experiments has been to gain a better understanding of the molecular mechanisms initiating the autoimmune process in type 1 diabetes. It has previously been established by the inventor that the early appearance of IAA (E-IAA) is a prediabetes marker. The age of 5 weeks in the NOD mouse corresponds to prior or around the first phase of the disease i.e. prior the presence of peri-insulitis or non-destructive insulitis. It has been assessed whether specific gene transcripts involved in known pathways are changed in the Pancreatic Lymph Nodes according to the presence or absence of E-IAA.

Between the genes found to be differentially expressed (IAA positive versus IAA negative samples) 32 genes code for potentially secreted proteins as assigned by Gene Ontology annotations and cellular localization. The majority of these genes have human orthologs.

The identified genes have the potential to be used as biological markers for autoimmune diseases such as type I diabetes (ex Reg 1, Reg2, Cel, Cckar, Spp1), multiple sclerosis (ex Spp1), collagen induced arthritis (ex Spp1), and autoimmune hepatitis (Spp1) as well as other genetic diseases such as cystic fibrosis (ex Ela1). Additional applications include diagnostic and prognostic markers for certain cancers and infection-mediated immune responses.

In addition to the potential of encoded proteins to be used as predictive biomarkers for early pre-inflammatory condition in autoimmune diseases (type 1 diabetes), they probably represent functional properties for pancreatic tissue damage and regeneration (Reg genes products and Spp1) and they are therefore valuable therapeutic targets.

### Materials and Methods

***Mice*** have been purchased from Taconic farms (NOD/*tac*), Germantown, NY, and housed in specific pathogen-free conditions. Experimental protocols were conformed to guidelines from the Institutional Animal Care and Use Committee. Pregnant females are tested one week before delivery for the presence of IAA.

Animals were tested for the presence of IAA as previously described {Melanitou, 2004}. Briefly, sera from 3 weeks old (at weaning) NOD mice and at 4 and 5 weeks have been assessed for the presence of IAA. The last sera for IAA detection are taken at 5 weeks of age on the moment of sacrificing the animals for the RNA preparation and represent another checkpoint for the presence or absence of IAA. The established phenotypic requirement dictates that the presence of IAA, when detected, will be confirmed at least twice within one-week intervals. Animals remaining IAA positive for two consecutive essays together with their IAA negative controls are sacrificed by cervical dislocation after light anesthesia. Pancreata are taken and separated in half, one sample for RNA preparation and the other for histology. This is not the case for the PaLN with which, due to its small size, one sample can be used for RNA or for histology and not for both.

**IAA assay:** IAA are measured in a previously established radioimmunoassay incorporating competition with unlabeled insulin and precipitation with Protein A/G sepharose in a 96 well filtration plate, as previously described {Melanitou, 2004}.

**RNA preparation for microarrays:** PaLN have been immediately placed in RNA later (Qiagen) and processed for total RNA preparation on the same day. Total RNAs were prepared with the Qiagen mini- or midi-RNA kit following the manufacturer's protocol. After quantification by spectrophotometry, 500 ng of Total-RNA was assessed for quality using an Agilent Bioanalyzer (Agilent Technologies). Integrity is evaluated by the presence of the ribosomal RNAs that should not be degraded.

High quality RNA samples (4.5 µg) from E-IAA positive (4 mice) and E-IAA negative (5 mice) were reversed transcribed and after biotinylated labeling of cRNA were used to hybridize to Affymetrix (Santa Clara, CA) first array MG-U74Av2 GeneChip according to standard Affymetrix protocols.

The first array in the set (MG-U74Av2) represents the majority of sequences (∼6,000) in the Mouse UniGene database that have been functionally characterized. In addition, approximately 6,000 EST clusters are also represented on this single array. In total MG-U74AV2 GeneChip contains probe sets representing 12,488 transcripts. MicroChip scanning and data capture were also according to standard Affymetrix methods:
(hftp://www.affymetrix.com/support/technical/manual/expression_manual.affx).

Normalization, filtering and sorting of the data was undertaken in Affymetrix Data Mining Tool software, preliminary explorative analyses were carried out in GeneSpring (Sillicon Genetics) and statistics assessed in Excel (Microsoft). For statistical analysis, data were log₂ transformed and since this was a first exploratory analysis, a Welch's T-test, which does not assume that variances are equal, with a p value cut-off =0.10 has been applied, to identify the most statistically significant changes in gene expression between E-IAA positive and negative samples. The Benjamini and Hochberg {Hochberg, 1990} false discovery rate as the multiple testing correction method has been used since this does not require equal number of samples in each group {Li, 2004}. Gene functions have been assessed with GO (Gene Ontology tool: http://www.geneontology.org).

### Data analysis:

### Data Analysis - Genespring GS6.0-II

Venn diagram representation of data from oligonucleotide micro array differential gene expression corresponding to IAA gene network. 9 samples have been used for positive (3+1) and negative sub (5 samples) phenotype. 1219 genes analyzed as follows:
1) calculate an estimate of noise, and find genes above the noise as follows gene expression must be called present in 4 of 9 samples.
2) Find genes that are "Present" in at least 4 of the 9 samples. Control signal above a given cut-off (45) and
3) Find genes that do not change either:
   a) Considering each sample separately (no grouping by phenotype) OR
   b) Grouping by phenotype

With regard to the "non-change" list: this list was generated by using the drawn gene function in which this artificial gene was a horizontal line. Then by finding genes with similar expression profile (p=0.90), genes have been hound in which there was no change in the levels across the experiment when each sample was taken individually. This is why carboxypeptidase H is there (P in all 9 samples) and raw expression values >1000, but is not in the list. It is so uniformly expressed that it got eliminated at this point.

Then this set of genes has been analyzed for differences between IAAneg and IAApos samples with a Welch's T-test (does not assume variances are equal), set the p value cut-off to 0.10 (since this is more of exploratory analysis) and used the Benjamini and Hochberg false discovery rate as the multiple testing correction method. This test has been used rather than the Wilcoxson Rank Sign test because that test requires equal number of samples in each group which was not the case in this experiment. Set up like this approximately 10% of the resulting "significant" genes could be chosen by accident. This test returned 125 genes.

**Scatter plot Arrays MU74AVersion 2, GS6.0.** Correlation of the expression of all genes for IAA positive *versus* IAA negative samples in PaLN has been plotted.

**Gene expression clustering**. Phenotype not considered.

Dendrogram based on the 1219 genes has been established. It is to be noted that there is not much obvious clustering of the genes that is consistent with, in either the IAA+ or IAA- , with the exception of the group in the frame line.

It is also to be noted that, in analyzing the figure obtained with the present data, one mouse that although has been E-IAA negative between 3 and 5 weeks of age, is placed by the clustering program together with the E-IAA positive samples.

**Representation of statistical analysis of the transcriptome data:** The 1219 genes have been analyzed for differences between IAAneg and IAApos samples with a Welch's T-test (does not assume variances are equal), set the p value cut-off to 0.10 (since this is more of exploratory analysis) and used the Benjamini and Hochberg false discovery rate as the multiple testing correction method. This test has been used rather than the Wilcoxson Rank Sign test because that test requires equal number of samples in each group and that was not the case in the present test. Set up like this approximately 10% of the resulting "significant" genes could be chosen by accident. This test returned **125 genes**.

**Quality control of data:** How many islet-specific genes are found also expressed in the PaLN? The expression patterns in all PaLN samples used (9 samples) of known pancreas-specific genes and present in the chip have been evaluated. Genes tested are: Somatostatin, Glucagon, Somatostatin Receptor, Islet Amyloid Polypeptide (IAPP) and insulin genes. It is interesting to notice that all pancreas-specific genes tested are not found to be expressed in the lymph nodes with the exception of the insulin genes that have been found to be expressed in some of the E-IAA positive samples. This is a quality control experiment of the tissue used in the arrays.

Chromosomal location of upregulated genes in the PaLN: Genes identified by the present inventor seem to cluster in single chromosomal loci, especially on chromosomes 3, 6, 7, 8 and 17, indicating a possible co-regulatory mechanism during transcription.

In order to improve the robustness of the present results, the data obtained from hybridization of the microchips have been analyzed with two additional softwares:
- DNA Analyzer dChip (http://www.dchip.org/) and
- the S+ Module S+ArrayAnalyzer, lnsighffull (www.insightful.com)

### Results and Discussion

### A. Phenotypic markers of early autoimmunity

Autoimmune diseases initiate when tolerance to self-antigens brakes down, a phenomenon that has been an intriguing question for immunologists in the 20^{th} century. During recent years an extensive body of literature has been published describing innovative methodologies for analyzing cellular and biochemical processes that characterize human autoimmune diseases on multiple levels. Nevertheless, the precise initiating events in the etiology of most human autoimmune diseases remain largely unexplained.

Studies have been undertaken by the inventor in order to identify an early phenotypic marker allowing the selection of individual mice as autoimmune prone, before any detectable autoimmune destruction appears. Consequently, the intent emerged to establish sub phenotypes that correlate with the final disease phenotype and thus simplify studies aiming to unravel the successive steps in T1D (type 1 diabetes) pathogenesis, as this disease constituted an example in the studies of human autoimmune pathology. This presented a challenge as albeit the NOD mouse model is an inbred strain, not all individuals develop the disease, with an incidence of 40-90% in female mice, depending on the colony {Leiter, 1993}.

The difficulty to select animals, similarly to human patients, at an early disease check point, prior to inflammation, has been bypassed in these experiments, by using the predictive value of Insulin Auto Antibodies (Report of the Expert Committee on the Diagnosis and Classification of Diabetes Mellitus, American Diabetes Association 1997 Diabetes Care 20, 1183-1197) and subsequently demonstrating the early appearance of IAA (E-IAA), and establishing it as a prediabetes marker {Melanitou, 2004}.

The inventor set up experiments to study this question and speculated that as early as at 3 weeks of age, at weaning, insulin autoantibodies (IAA) might be present in a subset of mice. Indeed it has been proposed that in the NOD mouse around this age (at weaning), autoimmune prone molecular changes might take place, before any insulitis (inflammation of the pancreas) is evident {André, 1996}.

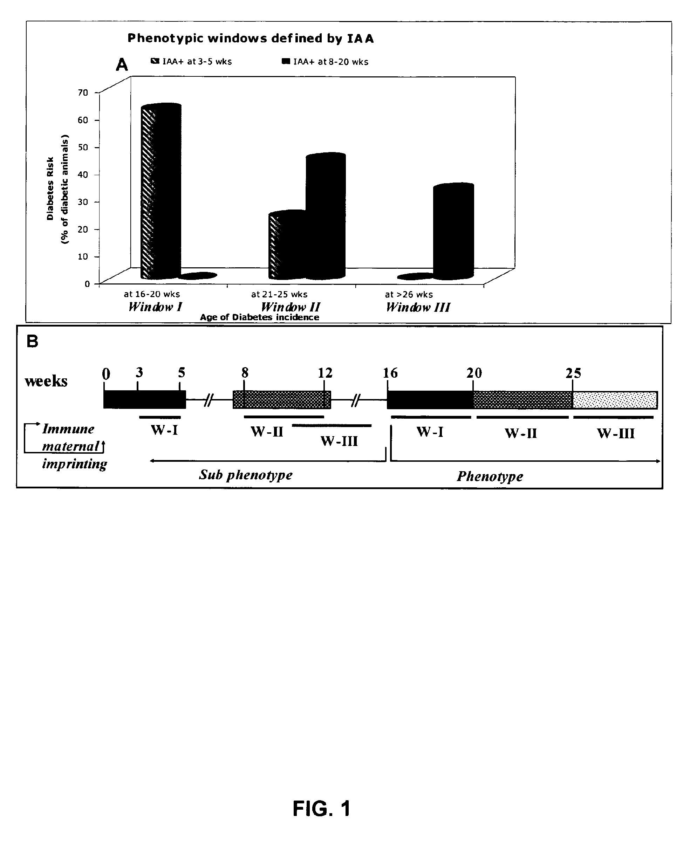

This work has produced some interesting results (Figure 1) and may be summarized as follows:
**1**. IAA can be detected as early as at 3 weeks of age in the NOD mice, before insulitis appears and it is a *quantal* phenotype as it runs by litters (Figure 1A). This indicates that the mechanisms of autoimmune destruction put in place early, are not only dependent upon genetic factors but also upon factors inherent to the individual animal/litter.
**2**. The maternal autoimmune-prone environment influences the IAA levels of the litters as reflected by the role played by maternal autoantibodies at *ante partum.* Indeed maternal autoantibodies influence the appearance of early autoimmunity in the litters (Figure 1B).
**3**. A biological role, etiologic or correlative, of Early-IAA has been found in later disease phenotype, thus establishing the early presence of insulin autoantibodies as an early marker of autoimmunity in the NOD mouse.
**4**. Finally, it was possible to establish three phenotypic "windows" corresponding to final disease onset and marked by the early presence of autoantibodies. **Window I** corresponds to early T1D onset at 16-20 weeks, **window II** to diabetes onset at 21-25 weeks and **window III** at 25-30 weeks of age (Figure 1A & 1B). These data show that the presence of E-IAA predisposes to early T1D and emphasize the biological significance of this sub phenotype as an early marker of autoimmunity {Melanitou, 2004}.

### B. Functional Genomics

The application of functional genomics implying high throughput differential gene expression analysis portrays with the possibility to decipher gene networks characteristic of specific biological processes, in carefully selected, by phenotypic attribution, samples.

Two types of tissues could be considered to be involved in autoimmune condition: **the target organ**, in the example of T1D the pancreas, whereas T cell destruction of the insulin producing β cells in the islets of Langerhans is taking place. Indeed, the target organ might be implicated in tissue autoimmune attack by the presence of tissue specific auto antigens, triggering auto reactive T and B cells and thus playing an integral part in the early events of diabetogenesis. However **immune tissues** seem also to play a role, as the immune system is unable to recognize self-antigens, negative selection of T cells in the thymus as well as apoptotic mechanisms of auto reactive immune cells are absent or deficient and therefore autoimmune destructive mechanisms are initiated.

Functional genomics studies were applied in immune tissues starting with the Pancreatic Lymph Nodes (PaLN) and the inventor assessed whether specific gene transcripts involved in known pathways are changed in this tissue, according to the presence or absence of E-IAA. It has been previously suggested that lymph nodes might be involved in early priming of T cells against pancreatic antigens {André, 1996}. In this possible scenario, p cell antigens might trigger reactive T cells to invade the islets. The hypothesis that gene expression changes might take place in a lymphoid tissue and in particular, in the closely to the pancreas located, PaLN was therefore considered.

For this aim, the early insulin autoantibodies sub phenotype has been used as an early marker of autoimmune destruction {Melanitou, 2004} to select individual mice, E-IAA positive or negative and apply genomic approaches. In total 9 individual NOD mice have been selected at 5 weeks of age and high quality T-RNAs have been prepared. The criteria for selecting these mice have been as follows: animals have been checked for IAA at 3, 4 and 5 weeks of age. Mice, at least twice E-IAA positive between 3 and 5 weeks of age, were used representing the autoimmunity positive samples (3 mice). Animals negative for E-IAA at the same ages constituted the negative controls (6 mice). It is however to be noted that one mouse, although E-IAA negative between 3 and 5 weeks of age, is place by the clustering program together with the E-IAA positive samples. With regarg to the expression profile, the groups were thus composed of 4 and 5 mice.

Affymetrix (Santa Clara, CA) oligonucleotide arrays were used to characterize the global patterns of transcriptional changes occurring between the two phenotypes. 125 genes out of the over 12,900 genes present in the microchip have been found to be differentially expressed between the two phenotypes, with 90 genes (72%) being up regulated in the RNA samples from E-IAA positive mice and 35 genes (28%) down regulated.

Between the genes found to be differentially expressed (IAApositive versus IAAnegative samples) 32 genes code for potentially secreted proteins as assigned by Gene Ontology annotations (*htt:*//*www.geneontology.org*/*)* and cellular localization (Table I). The majority of these genes have human orthologs (see for GeneBank assession numbers and description Table II).

### C. Expression patterns of genes coding for secreted proteins

As it has been described above, the transcriptome analysis of the pre-diabetes phenotypes in the NOD mouse revealed 125 genes showing statistically significant differences of gene expression between the 2 groups: the lAApos and the IAAneg. It is however to be noted that, in analyzing the present data, one mouse that although has been E-IAA negative between 3 and 5 weeks of age, is placed by the clustering program together with the E-IAA positive samples

Interestingly 40 of these 125 genes showed differences between the two groups of samples over 10 fold and reaching up to 122 fold. The inventor has assessed for the presence of genes coding for secreted proteins within this set of genes. Indeed 32 in total code for potential secreted proteins (Table II). From this set of genes, 25 belong to the 40 highly regulated genes and 7 are less regulated showing 3 to 8 fold differences between the two sets of samples (Table II).

**TABLE I : Statistics and differentially regulated expression patterns of genes coding for secreted proteins in PaLN of IAA pos and IAA neg NOD mice. Columns represent: Probe Set ID: gene identifier on the Affymetrix chip; Genbank: accession numbers of NCBI gene bank; Common name: gene symbol; Potential function: as found by in silico analysis; P-value: represents statistical significance of expression patterns during hybridization of the chip with cRNA probes; mean and StdDev: concerns the mean of expression values in all IAApos or IAAneg samples studied; Fold increase: IAApos vs IAAneg mean values.**

| | | | | | **IAA pos** | | **IAA neg** | | |
|---|---|---|---|---|---|---|---|---|---|
| **Probe Set ID** | **Genbank** | **Common Name** | **Potential function** | **P-value** | **mean** | **StdDev** | **mean** | **StdDev** | **Fold Increase** |
| 161890_f_at | AV371861 | Pap | Regeneration, T1D | 0,04490 | 5235,10 | 4992,72 | 40,72 | 26,07 | **129,00** |
| 96009_s_at | D63359 | Pap | Regeneration, T1D | 0,04247 | 15496,60 | 14076,85 | 149,36 | 76,13 | **104,00** |
| 103954_at | D63357 | Reg3a | Regeneration, T1D | 0,02618 | 6128,75 | 5230,56 | 65,60 | 33,15 | **93,00** |
| 95786_at | D14011 | Reg2 | Regeneration, T1D | 0,03315 | 19907,93 | 11545,00 | 224,24 | 336,31 | **89,00** |
| 94716_f_at | M17962 | Klk9 | | 0,01012 | 22111,73 | 12919,25 | 293,92 | 309,76 | **75,00** |
| 95509_at | AA607809 | Sycn | Inhibits insulin exocytosis | 0,02756 | 29455,68 | 10793,43 | 401,12 | 643,08 | **73,00** |
| 92601_at | AA674409 | Pnliprp1 | IL-4 inducible gene from cytotoxic T cells | 0,04781 | 27814,80 | 9681,68 | 471,90 | 924,04 | **59,00** |
| 100061_f_at | M13500 | Klk6; Kal; Klk1; mGk-6; 0610007D04Rik | | 0,01502 | 16742,50 | 7117,12 | 344,42 | 389,70 | **49,00** |
| 98041_at | X60103 | Rib1; Rib-1; AI574248 | Tumorigenesis | 0,04066 | 17303,93 | 10912,49 | 350,60 | 681,57 | **49,00** |
| 93302_at | U78770 | Tff2; SP; mSP | upregulated in epithelial damage, regeneration | 0,04066 | 15629,48 | 7126,60 | 377,46 | 714,00 | **41,00** |
| 92252_at | D85605 | Cckar; AW106902 | Potentiation of insulin secretion | 0,02284 | 1756,73 | 1272,46 | 52,26 | 40,42 | **34** |
| 162196_f_at | AV059956 | Itmap1 | pancreatitis | 0,03315 | 1990,95 | 1370,63 | 59,30 | 111,50 | **33,50** |
| 104495_f_at | Y00500 | Klk5; mGK-5 | | 0,01453 | 5064,75 | 2830,56 | 153,96 | 132,95 | **33,00** |
| 160132_at | AA710635 | Clps | body fat absorption lethal before winning | 0,06059 | 38519,88 | 6705,89 | 1266,48 | 2087,55 | **30,00** |
| 160070_at | M30687 | Pnliprp2 | IL-4 inducible gene from cytotoxic T cells | 0,02284 | 3144,58 | 1939,43 | 110,64 | 43,43 | **28,00** |
| 160213_at | D14010 | Reg1 | | 0,04781 | 45439,63 | 9625,48 | 1634,60 | 2846,75 | **28,00** |
| 101058_at | J00356 | Amy1 | | 0,02913 | 1838,23 | 1044,23 | 80,52 | 57,71 | **23,00** |
| 97519_at | X13986 | Spp1 | | 0,02284 | 685,80 | 340,80 | 34,34 | 31,18 | **20,00** |
| 160120_i_at | A1327450 | Pla2g1b | Oxydative injury, inflammation? | 0,00624 | 18849,17 | 5890,93 | 936,62 | 472,50 | **20** |
| 99939_at | U37386 | Cel | Tissue architecture | 0,06059 | 60131,43 | 10961,17 | 3151,22 | 5220,77 | **19,00** |
| 93783_at | M27347 | Ela1 | Secreted from macrophages or lymphocytes | 0,08202 | 57396,60 | 11908,46 | 3557,88 | 6132,38 | **16** |
| 160421_r_at | AA590358 | 2200008D09Rik | | 0,08202 | 80626,95 | 27075,77 | 5333,68 | 8914,60 | **15,00** |
| 94037_at | X04573 | Ela2 | | 0,08202 | 51033,10 | 13635,60 | 3440,44 | 6820,47 | **14,80** |
| 100103_f_at | AE000663 | Tcrb | immunologic synapse | 0,06059 | 50774,88 | 11042,98 | 3544,08 | 5681,64 | **14** |
| 102918_at | M84683 | Muc1 | overexpressed in carcinoma cells | 0,02284 | 2043,50 | 1064,87 | 156,36 | 113,91 | **13,00** |
| 93575_at | AF051102 | Ggh | | 0,09470 | 1723,50 | 1151,46 | 214,24 | 63,04 | **8,00** |
| 94392_f_at | U22516 | Ang | | 0,02284 | 455,08 | 180,56 | 74,92 | 28,92 | **6,00** |
| 97964_at | AW122851 | 1110002023Rik | | 0,04259 | 1190,78 | 489,14 | 210,82 | 60,58 | **5,60** |
| 95137_at | A1852985 | 1810014L12Rik | | 0,04414 | 2097,25 | 986,20 | 394,26 | 130,10 | **5,00** |
| 162276_i_at | AV367855 | C1qb | | | 915,43 | 625,86 | 225,70 | 76,91 | **4,00** |
| 102197_at | AJ222586 | Nucb2 | | | 767,60 | 337,49 | 273,18 | 83,82 | **3,00** |
| 98549_at | M77123 | Vtn | | 0,02284 | 2040,65 | 518,20 | 767,72 | 223,07 | **3,00** |

**TABLE II: Genes coding for secreted proteins in PaLN of NOD mice.**

| | **Affymetrix Location** | **Common Name** | **Genbank** | **Fold Inc.** | **Description** | **Potential function** |
|---|---|---|---|---|---|---|
| 1 | 161890_f_at | Pap | AV371861 | 129,00 | pancreatitis-associated protein, acute-phase response inflammatory response | Regeneration, T1D |
| 2 | 96009_s_at | Pap | D63359 | 104,00 | pancreatitis-associated protein | Regeneration, T1D |
| 3 | 103954_at | Reg3a | D63357 | 93,00 | regenerating islet-derived 3 alpha, acute-phase response inflammatory response | Regeneration, T1D |
| 4 | 95786_at | Reg2 | D14011 | 89,00 | regenerating islet-derived 2 | Regeneration, T1D |
| 5 | 94716_f_at | Klk9 | M17962 | 75,00 | kallikrein 9, kallikrein 6, kallikrein 1, nerve growth factor, alpha, RIKEN cDNA 1700127D06 gene | |
| 6 | 95509_at | Sycn | AA607809 | 73,00 | syncollin | Inhibits insulin exocytosis |
| 7 | 92601_at | Pnliprp1 | AA674409 | 59,00 | pancreatic lipase related protein 1 | |
| 8 | 100061_f_at | Klk6; Kal; KIk1; mGk-6; 0610007D04Rik | M13500 | 49,00 | kallikrein 6 | |
| 9 | 98041_at | Rib1; Rib-1; AI574248 | X60103 | 49,00 | ribonuclease, RNase A family, 1 | Tumorigenesis |
| 10 | 93302 at | Tff2; SP; mSP | U78770 | 41,00 | trefoil factor 2 (spasmolytic protein 1) | upregulated in epithelial damage, regeneration? |
| 11 | 92252_at | Cckar; AW106902 | D85605 | 34 | cholecystokinin A receptor | Potentiation of insulin secretion |
| 12 | 162196_f_at | Itmap1 | AV059956 | 33,50 | Mus musculus integral membrane-associated protein 1 | |
| 13 | 104495_f_at | Klk5; mGK-5 | Y00500 | 33,00 | kallikrein 5 | |
| 14 | 160132_at | Clps | AA710635 | 30,00 | colipase, pancreatic | |
| 15 | 160070 at | Pnliprp2 | M30687 | 28,00 | pancreatic lipase-related protein 2 | IL-4 inducible gene from cytotoxic T cells |
| 16 | 160213_at | Reg1 | D14010 | 28,00 | regenerating islet-derived 1 | |
| 17 | 101058_at | Amy1 | J00356 | 23,00 | amylase 1, salivary | |
| 18 | 97519_at | Spp1 | X13986 | 20,00 | secreted phosphoprotein 1, ossification cell adhesion, cytokine activity protein binding, TGF Beta Signaling Pathway | |
| 19 | 160120_i_at | Pla2g1b | AI327450 | 20 | phospholipase A2, group IB, response to stress cell proliferation phospholipid catabolism lipid catabolism | Oxydative injury, inflammation |
| 20 | 99939_at | Cel | U37386 | 19,00 | carboxyl ester lipase | Tissue architecture |
| 21 | 93783_at | Ela1 | M27347 | 16 | elastase 1 | Secreted from macrophages or lymphocytes |
| 22 | 160421_r_at | 2200008D09Rik | AA590358 | 15,00 | | |
| 23 | 94037_at | Ela2 | X04573 | 14,80 | proteolysis and peptidolysis | |
| 24 | 100103_f_at | Tcrb | AE000663 | 14 | | immunologic synapse |
| 25 | 102918_at | Muc1 | M84683 | 13,00 | mucin 1, transmembrane | overexpressed in carcinoma cells |
| 26 | 93575_at | Ggh | AF051102 | 8,00 | gamma-glutamyl hydrolase | |
| 27 | 94392_f_at | Ang | U22516 | 6,00 | angiogenin, ribonuclease A family, member 1, protein biosynthesis cell differentiation | |
| 28 | 97964_at | 1110002O23Rik | AW122851 | 5,60 | FK506 binding protein 11, protein folding | |
| 29 | 95137_at | 1810014L12Rik | AI852985 | 5,00 | | |
| 30 | 162276_i_at | C1qb | AV367855 | 4,00 | complement component 1, q subcomponent, beta polypeptide, Complement_Activation_Classical pathway | |
| 31 | 102197_at | Nucb2 | AJ222586 | 3,00 | nucleobindin 2, calcium ion homeostasis | |
| 32 | 98549_at | Vtn | M77123 | 3,00 | vitronectin, cell adhesion cell-matrix adhesion, Inflammatory Response Pathway | |

### TABLE II

### D. Gene classification according to functional annotation

Secreted proteins are found in the extracellular space and represent the major class of molecules implicated in intercellular communication in multicellular organisms. In the mouse this class of proteins is referred to as the mouse "secretome" {Greenbaum, 2001}.

Aiming to a better understanding of the implication of these genes in autoimmune diabetes in particular and in autoimmune diseases in general, searches were carried out *in silico* for functional annotations concerning these genes. With the exception of 3 of those corresponding to ESTs (Expressed Sequence Tags) and probably representing new, or similar to known, genes, the 29 remaining code for known proteins.

It was thus possible to classify the identified genes according to their function in 4 groups (Table III).

**TABLE III: Classification according to function of secreted proteins coding genes**

| **Group** | **Function** | **N° of genes** |
|---|---|---|
| **I** | Tissue regeneration & integrity | 8 +*Spp1* |
| **II** | Tumorigenesis | 9 +*Spp*1 |
| **III** | Immune function | 9 +*Spp*1 |
| **IV** | Unclassified | 5 |

***TABLES IV:*** Statistics, annotations and references of biological function of classified genes coding for secreted proteins. **a: Group 1:** Tissue integrity and regeneration. **b. Group 2:** Tumorigenesis. **c**. **Group 3**: Immune function. **d. Group 4:** Unclassified genes

**Table IVa: Tissue integrity and regeneration**

| **Probe Set ID** | **Genbank** | **Common Name** | **Disease** | **Potential function** | **P-value** | **Fold Increase** |
|---|---|---|---|---|---|---|
| 161890_f_at | AV371861 | Pap; pancreatic associated protein | Hepatocellular carcinoma, pancreatitis | Regeneration, T1D | 0,04490 | 129,00 |
| 96009_s_at | D63359 | Pap | | Regeneration, T1D | 0,04247 | 104,00 |
| 103954_at | D63357 | Reg3a; Regenerating protein | | Regeneration, T1D | 0,02618 | 93,00 |
| 95786_at | D14011 | Reg2 | | Regeneration, T1D | 0,03315 | 89,00 |
| 99939_at | U37386 | Cel; carboxyl ester lipase | Type 1 diabetes | Tissue architecture | 0,06059 | 19,00 |
| 160213_at | D14010 | Reg1 | | Regeneration, T1D | 0,04781 | 28,00 |
| 93302_at | U78770 | Tff2; SP; mSP; trefoil factor 2 (spasmolytic protein 1) | Gastrointestinal tract | upregulated in epithelial damage, regeneration | 0,04066 | 41,00 |
| 160132_at | AA710635 | Clps; colipase, pancreatic | Type 2 diabetes | body fat absorption lethal before winning | 0,06059 | 30,00 |
| 97519_at | X13986 | Spp1; osteopontin | renal allograft rejection | Pleiotropic with several functions | 0,02284 | 20,00 |

**Table IVb: Tumorigenesis**

| **Probe Set ID** | **Genbank** | **Common Name** | **Disease** | **Potential function** | **P-value** | **Fold Inc.** |
|---|---|---|---|---|---|---|
| 94716_f_at | M17962 | KIk9 | Prostate cancer | | 0,01012 | 75,00 |
| 100061_f_at | M13500 | KIk6; Kal; KIk1; mGk-6; 0610007D04Rik | breast cancer & prostate carcinomas & salivary gland carcinomas | | 0,01502 | 49,00 |
| 98041_at | X60103 | Rib1; Rib-1; AI574248 | | | 0,04066 | 49 |
| 104495_f_at | Y00500 | Klk5; mGK-5 | Prostate cancer | | 0,01453 | 33,00 |
| 102918_at | M84683 | Muc1, Mucin or episialin | Gastric carcinoma | overexpressed in gastric carcinoma cells | 0,02284 | 13,00 |
| 92252_at | D85605 | Cckar; Cholecystkinin receptor | Colon and pancreatic cancer | Potentiation of insulin secretion | 0,02284 | 34 |
| 93575_at | AF051102 | Ggh; gamma glutamyl hydrolase | cancer; pulmonary tumors | | 0,09470 | 8,00 |
| 94392_f_at | U22516 | Ang; angiogenin | Pancreatic cancer; multiple myeloma; neroendocrin carcinoma | Notch signaling; angiogenesis, metastasis | 0,02284 | 6,00 |
| 102197_at | AJ222586 | Nucb2; nucleobindin 2 | B-cell lymphomas | DNA-binding protein NEFA precursor; calcium ion homeostasis | | 3,00 |
| 97519_at | X13986 | Spp1 | Bone tumors | metastatic; cell adhesion | 0,02284 | 20,00 |

**Table IVc: Immune function**

| **Probe Set ID** | **Genbank** | **Common Name** | **Potential function** | **Disease** | **P-value** | **Fold Inc.** |
|---|---|---|---|---|---|---|
| 160070_at | M30687 | Pnliprp2; pancreatic lipase-related protein 2 | IL-4 inducible gene from cytotoxic T cells | lipid catabolism; lipid metabolism; cellular defense response | 0,02284 | 28,00 |
| 160120_i_at | A1327450 | Pla2g1b; phospholipase A2, group IB, pancreas | Oxydative injury, inflammation Antiinflammatory molecule | lipid catabolism, stress response, phospholipid catabolism, cell proliferation | 0,00624 | 20 |
| 93783_at | M27347 | Ela1; elastase 1, pancreatic | Secreted from macrophages or lymphocytes | arthritis; asthma: lung disease, cystic fibrosis | 0,08202 | 16 |
| 94037_at | X04573 | Ela2; elastase 2 | proteolysis and peptidolysis | Type 1 diabetes, Blood disease | 0,08202 | 14,80 |
| 100103_f_at | AE000663 | UKNOWN within Tcrb locus | immunologic synapse | | 0,06059 | 14 |
| 92601_at | AA674409 | Pnliprp1; pancreatic lipase related protein 1 | IL-4 inducible gene from cytotoxic T cells | infectious diarhea | 0,04781 | 59,00 |
| 162196_f_at | AV059956 | Itmap1; ntegral membrane-associated protein 1 ;CUB and zona pellucida-like domains 1 | pancreatitis | | 0,03315 | 33,50 |
| 98549_at | M77123 | Vtn; vitronectin | Inflammatory response pathway, cell adhesion | Hepatocellular carcinoma | 0,02284 | 3,00 |
| 162276_i_at | AV367855 | C1qb | complement component 1, q-subcomponent | Potential b-defensin homologue | | 4,00 |
| 97519_at | X13986 | Spp1 | Pleiotropic with several functions, cell adhesion | T cell polarization T-beta dependent | 0,02284 | 20,00 |

**Table IVd: unclassified genes**

| **Probe Set ID** | **Genbank** | **Common Name** | **Potential function** | **P-value** | **Fold Increase** |
|---|---|---|---|---|---|
| 95509_at | AA607809 | Sycn; syncollin | Inhibits insulin exocytosis | 0,02756 | 73,00 |
| 101058_at | J00356 | Amy1; amylase 1, salivary | carbohydrate metabolism | 0,02913 | 23,00 |
| 160421_r_at | AA590358 | 2200008D09Rik; Ctrb1; chymotrypsinogen B1 | UKNOWN; Chronic pancreatitis | 0,08202 | 15,00 |
| 97964_at | AW122851 | 1110002O23Rik | UKNOWN annotated FK506 binding protein 11, Signaling enzyme in T cell activation | 0,04259 | 5,60 |
| 95137_at | Al852985 | 1810014L12Rik | UKNOWN; human orthologue found in meningiomas | 0,04414 | 5,00 |

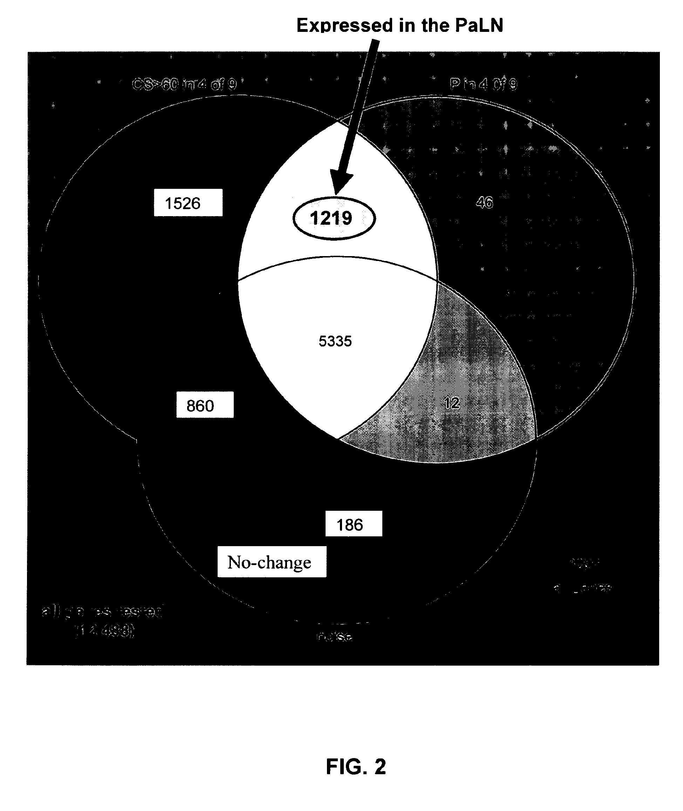

Spp1 codes for the secreted phosphoprotein named also osteopontin (opn), or Eta1 gene, for Early T lymphocyte activation protein. Spp1 has pleiotropic functions (Figure 2) and it has been proposed to play a role in various inflammatory diseases such as rheumatoid arthritis {Yamamoto, 2003}, bone tumors {Natasha, 2006}, multiple sclerosis {Chabas, 2001}, bacterial infections {Patarca, 1993}, as well as it may act as a cytokine and promote T cell polarization and macrophage migration {Ashkar, 2000}. Therefore Spp1 is part of all groups of secreted proteins (Table III & IV).

In group 1 (Table IVa) are placed 8 genes that are implicated in tissue integrity and regeneration. Five of these genes code for a set of related proteins named Pap for pancreatic-associated proteins or Reg for regenerating proteins and they have been found to play a role in tissue regeneration {Yuan *et al,* 2005 and reviewed by Graf, 2005}. It has been recently reported that adult human islets possess a remarkable degree of morphogenetic plasticity {Jamal, 2005}. This observation might represent a new tool in the understanding of pancreatic carcinogenesis and islet neogenesis, a phenomenon that could contribute also in diabetes therapy. Moreover, islet neogenesis and tissue preservation by inherent factors could be a valuable mechanism against β-cell destruction. Proteins playing a role in tissue integrity and regeneration might contribute in these processes.

In the same group are placed the *Tff*'2 gene coding for the Trefoil factor 2, called also spasmolytic peptide and the gene coding for colipase pancreatic (Clps). *Tff'*2 has been found to be expressed in the gastrointestinal tract and it is up regulated in epithelial damage {Tomasetto, 1990; Thim, 2005;

Baus-Loncar, 2005; Dhar, 2005}, with a potential role in tissue regeneration. *Clps* have been recently found to be implicated in type 2 diabetes {Lindner, 2005}. Finally, in the Group 1 is also placed the gene coding for the carboxyl ester lipase (*Cel*). It has been very recently reported that mutations found in the VNTR (variable number of tandem repeats) within exon 11 of the *Cel* gene cause a diabetes syndrome and exocrine pancreas dysfunction {Raeder, 2006}. This is a very interesting result suggesting a cross talk between exocrine and endocrine cells of the pancreas and opening a new challenge in the understanding of diabetes. This is in accordance with the data reported herein, indicating that genes with diverse functions and abnormally expressed at specific early disease checkpoints, might be implicated in disease by disturbing an established cellular steady state and therefore create the initiation of pathogenic events. The levels of expression in fold increase, of the genes in Group I are shown schematically in Figure 4.

In group II (Table IVb) are placed genes that have been reported to be implicated in tumor formation.

Thus 3 genes coding for kallikreins (*Klk*9*, Klk*6 or *Klk*1 and *Klk*5) have been found to be differentially expressed in this experimental design (Figure 5).

Tissue kallikreins are serine proteases with a high degree of tissue specificity, thought to be involved in the generation of bioactive peptides, the kinines, in many organs, such as the kidney, salivary glands, pancreas and blood vessels. They constitute a large multigene family highly similar between humans and rodents {Yousef, 2000}. *Klk6* has a region showing homology with the protease serine 1 (PRSS1) (OMIM 276000). The PRSS9 mRNA was expressed in several primary tumors and cell lines from mammary, prostate, and ovarian cancers, but was not detected in any metastases of these cancers {Stamey, 1987}.

The mucin *(Muc 1*) gene or episialin has been found to be highly expressed in gastric carcinoma and proposed to be involved in gastric carcinogenesis {Silva, 2001}. Similarly the genes coding for Cholecystkinin (*Cckar*), gamma glutamyl hydrolase (*Ggh*) and angiogenin (*Ang*) have been found implicated in the formation of various tumors {Takata, 1997; Cheng, 2005; Esaki, 1998; He, 2004; Bond, 1990}. Angiogenin in particular is involved in the Notch signaling pathway related to neurovascular progression of pancreatic cancer {Buchler, 2005} and metastasis, as well as in multiple myeloma {Politou, 2005}. It has been reported that circulating levels of angiogenic cytokines can predict tumor progression and prognosis in neuroendocrin carcinomas of the gastro-entero-pancreatic system {Pavel, 2005}. The authors reported that specific anti-angiogenic therapies should be evaluated in neuroendocrine carcinoma patients {Pavel, 2005}.

Finally, the gene coding for nucleobindin 2 *(Nucb2)* is implicated in B cell lymphomas {de Vos, 2003}.

It is a DNA binding protein called also NEFA and its sequence contains a signal peptide suggesting that it is a secreted or transmembrane protein.

Three of the genes (the *Ggh, Ang* and *Nucb2*) in the Tumorigenesis group (Group II) showed a lower differential expression, in this experimental design, ranging from 3 to 8 fold of increase between the

IAA pos and IAA neg groups of animals (Table IVb and Figure 5). They have been however placed in the set of secreted genes due to their identification by two different methods of array data analysis (GeneSpring and GeneShifter softwares).

The relevance of the presence of genes implicated in tumorigenesis in this data set can be as follows:

The events leading in autoimmune destruction might share common characteristics with several inflammatory conditions leading in cancer as well as in autoimmunity.

In the third group are placed genes coding for secreted proteins found to have some immune function (Table IVc, Group III). Interestingly, three genes coding for lipase proteins and two for elastase have been placed in this group (see Figure 6 for fold of gene expression).

Pancreatic lipase related protein 2 *(Pnliprp2)* is expressed in cytotoxic T cells and it is an IL-4 inducible gene, playing a role in cellular defense response {Grusby, 1990}. Phospholipase A2 group 1B (*Pla2g1b*) is an anti-inflammatory molecule implicated in oxidative stress and cell proliferation {Snyder, 1999}. Pancreatic lipase related protein 1 (*Pnliprp*1) has been found in acute pancreatitis with an infectious pathogens etiology {Reimund, 2005}.

Elastase 1 (*Ela*1) is an enzyme secreted by macrophages or lymphocytes {Yamasaki, 1987}. It is a pro inflammatory protein and has been found to be related in adjuvant induced arthritis {Escandell, 2006}, asthma, lung disease and cystic fibrosis {Bines, 2005}.

The integral membrane-associated protein 1 (*Itmap*) contains a CUB domain and plays an essential role in trypsinogen activation and both impaired and augmented trypsinogen activation can be associated with protection of increased severity of pancreatitis {Imamura, 2002}. The CUB domain is an extracellular domain of approximately 110 residues which is found in functionally diverse, mostly developmentally regulated proteins. Almost all CUB domains contain four conserved cysteines which probably form two disulfide bridges (C1-C2, C3-C4). The structure of the CUB domain has been predicted to be a β-barrel, similar to that of immunoglobulins. It is interesting to note that two of the genes identified in these microarray experiments contain the CUB domain or a zinc metalloprotease domain: the *Itmap* and the complement subcomponent *C1q.* The biological significance of the CUB domain in these proteins remains to be elucidated.

Finally two genes showed a lower level of differential gene expression in this experimental design: the *Vtn* and *C1qb* (Figure 6). The vitronectin *(Vtn)* coding gene is a cell adhesion molecule involved in the pathway of inflammatory response and expressed in hepatocytes {Seiffert, 1991}. Vitronectin together with metalloproteases have been found to increase in the serum of patients with hepatocellular carcinoma {Paradis, 2005}.

The Complement component 1, q subcomponent (*C1qb*) gene is a beta-defensin homologue and its isoforms have been found to cooperatively contribute to innate immunity in the urogenital system {Yamaguchi, 2002}. Finally one unknown gene (Genbank accession number: AE000663) codes for a protein possessing a signal peptide and is located within the T cell receptor beta *(Tcrb)* locus. It is an unknown protein and due to similarity with the Tcrb segments, it has been suggested to play a role in the immunologic synapse.

In the last group are placed proteins coded by three genes of unknown function represented by ESTs (Figure 7).

Interestingly, one of these genes (Genbank accession number: AW122851) has been very recently annotated to have homology with the FK506 binding protein 11, an immunophillin, a signaling enzyme in T cell activation {Price, 2005}. It has been also reported as a novel target of immunosuppressive agents as found by microarrays analysis {Cristillo, 2002}. Immunosuppressive drugs such as cyclosporine A and FK506 both interfere with Ca(2+)-sensitive T-cell signal transduction pathway, thereby preventing the activation of specific transcription factors (such as NF-AT and NF-IL2A) involved in lymphokine gene expression. These drugs act by interaction with their cognate intracellular receptors, cyclophilin and FK506 Binding Protein, respectively. The authors reported that over expression of calcineurin in Jurkat cells renders them more resistant to the effects of immunosuppressive drugs (FK506). Jurkat cell line has derived from human T-cell leukemia and used to determine the mechanism of differential susceptibility to anti-cancer drugs and radiation. Calcineurin has been identified as a key enzyme in the T-cell signal transduction cascade and biological evidence has been provided to support the notion that the interaction of drug-isomerase complexes with calcineurin underlies the molecular basis of CsA/FK506-mediated immunosuppression.

Other genes placed in this group are the amylase (Amy 1) and the Syncollin (Sycn). This later gene has been recently found to inhibit insulin exocytosis {Wasle, 2005}. The amylase coding gene could have an immune function because of its implication in inflammatory syndromes {Chen *et al,* 2005; Pinelli *et al,* 2006}.

### Summary and Perspectives:

The nature of the immune response in various pathophysiological conditions is determined by the dynamic interaction of cells of the immune system with other cells, antigens and secreted factors.

Understanding the complexity of such responses under physiological or pathological conditions might contribute to the comprehension of the origin of several common complex disorders in which the immune system plays the leading role.

In view of the multitude of molecular interactions taking place in such disease processes, governed by a multitude of genes, global approaches represent a commendable way to go towards gene identification. Transcriptome offers the possibility to assess simultaneously several thousands of genes.

The observations made from the genes coding for secreted proteins identified in this work consent to advance the following hypotheses:
**1. Tissue architecture** might be in the core of the initial steps of autoimmune destruction as well as of other immune responses as it is observed in the case of tumorigenesis. In this case the transformed cell escapes recognition by the immune system and by proliferating allows transforming tissue architecture. Similarly but opposing to tumorigenesis, in the case of autoimmune destruction **tissue integrity** is also attained by the immune destructive lymphocytes. Therefore proteins involved in tissue regeneration and integrity might play a key role in these diseases and they represent potential diagnostic tools and therapeutic targets.
2. Similarly, the genes found in this transcriptome data to have a somehow collective **immune function** role might have a **universal task** in the initiation of various conditions including cancer, infection and autoimmunity. After initiating the disease, other genetic factors might take over the disease specific steps, characteristic of the particular clinical signs.
3. Finally, the presence of **pleiotropic** proteins (see *Spp*1 or osteopontin) in these data argues in favor of all three hypotheses and underlines the possibility of a **common origin** in diseases as different as autoimmunity, cancer and infection. It is noteworthy that Spp1 is highly up regulated in the diabetes-prone NOD mice, prior to any clinical sign.

It can be advanced the concept that tumorigenesis is the reverse process of autoimmunity as far as the involvement of the immune system is concerned. In tumor formation the immune system is inactive or inoperative since incapable to provide proper protection of the organ against the tumor cells. In contrast during the autoimmune process the immune system is over activated and attacks the self tissue. While in both situations the reverse actions should take place if a physiological response has to be considered.

The genes identified in these experiments might represent not only diagnostic molecules and therapeutic targets, but also experimental tools for deciphering the validity of these hypotheses.

### Example 2: Human ortholgs and chromosomal localization

The following orthologs are the orthologs of the mouse proteins recited in example 1.

| **Genbank** | **Common Name** | **Human ortholog** | **Human chr locus** | **Mus chr locus** |
|---|---|---|---|---|
| AV371861 | Pap | NM 138938; NM 138937 Regenerating islet-derived 3 alpha (REG3A), transcript variant 2 | | 6 C |
| D63359 | Pap | NM 002580 Regenerating islet-derived 3 alpha (REG3A), transcript variant 1 | 2p12 | 6 C |
| D63357 | Reg3a | AA399061 Pancreatitis-associated protein 1 (PAP) | 2p12 | 6 33.5 cM |
| D14011 | Reg2 | D17291; NM 006507 Regenerating protein I beta | 2p12 | 6 C3 |
| M17962 | Klk9 kallikrein 9 and in human: kallikrein 6 kallikrein 1 nerve growth factor, alpha RIKEN cDNA 1700127D06 gene | AF188747; S39329 and human variants: NM 010116 NCBI Mus musculus kallikrein 9 (Klk9), mRNA NM 010645 NCBI Mus musculus kallikrein 1 (Klk1), mRNA NM 010639 NCBI Mus musculus kallikrein 6 (Klk6), mRNA NM 010915 NCBI Mus musculus nerve growth factor, alpha (Ngfa), mRNA NM 029831 NCBI Mus musculus RIKEN cDNA 1700127D06 gene (1700127D06Rik), mRNA. Prostate kallikrein 2 (KLK2) | 19q13.41 | 7 23.18 cM |
| AA607809 | itmap1 | XM 371167 Syncollin (SCN) or Integral membrane protein like 1 | 19q13 | 7 A3 |
| AA674409 | Pnliprp1 | NM 006229 Pancreatic lipase-related protein 1 (PNLIPRP1) | 10q25.3 | 19 29.0 cM |
| M13500 | Klk6; Kal; Klk1; mGk-6; 0610007D04Rik | DY321134 Putative; NP 002248.1 65% homology Kallikrein 6 (KLK6) | | 7 23.0 cM |
| X60103 | Rib1; Rib-1; Al574248 | NM 002933; PubMed ID : 10393896 for tumor implication Pancreatic ribonuclease RNase A family 1 (RNASE1) | 14q11.2 | 14 18.5 cM |
| U78770 | Tff2; SP; mSP | X51698. Trefoil factor 2 or spasmolytic protein1 or Spasmolytic polypeptide (SP) | 21q22.3 | 17 17.0 cM |
| D85605 | Cckar; AW106902 | L13605. cholecystokinin A receptor (CCKAR) | Location 4p15.1-p15.2 | 5 34.0 cM |
| AV059956 | Itmap1 | NM 022034. CUB and zona pellucida-like domains 1 (CUZD1 or ERG-1) (estrogen regulated gene 1) | 10q26.13 | 7 F3 |
| Y00500 | Klk5; mGK-5 | No human orthologue | None | 7 23.01 cM |
| AA710635 | Clps | NM 001832. Pancreatic colipase (CLPS) | 6pter-p21.1 | 17 17.1 cM |
| M30687 | Pnliprp2 | T29248; NM 005396 pancreatic lipase-related protein 2 | 10q25.3 | 19 D3 |
| D14010 | Reg1 | AF172331 lithostathine (REG1A) or islet Regenerating | 2p12 | 6 33.5 cM |
| J00356 | Amy1 | A1539849; NM 004038 Amylase 1 (or alpha-amylase 2B precursor) | 1 p21 | 3 50.0 cM |
| X13986 | Spp1 | J04765 secreted phosphoprotein 1 or osteopontin (OPN) | 4q21-q25 | 5 56.0 cM |
| AI327450 | Pla2g1b | NM 000928 phospholipase A2, group IB (pancreas) | 12q23-q24.1 | 5 F1-G1.1 |
| U37386 | Cel | NM 001807 Carboxyl ester lipase (bile salt-stimulated lipase) (CEL) | 9q34.3 | 2 16.0 cM |
| M27347 | Ela1 | AF120493 Elastase 1 (ELA1) or Elastase 1 precursor | 12q13 | 15 56.8 cM |
| AA590358 | 2200008D09Rik; Ctrb1 | NM 001906 chymotrypsinogen B1 (CTRB1) | 16q23-q24.1 | 8 57.0 cM |
| X04573 | Ela2 | M16652 IIA mRNA homology with human ELA1 gene pancreatic elastase IIA | 1p36.21 | 4 E1 |
| AE000663 | Tcrb; PRSS3 | NM 002771; AF009664 for TCRb human protease, serine, 3 (mesotrypsin) (PRSS3) | 9p11.2; 7q34 | 6 B1 |
| M84683 | Muc1 | NM 002456 Transmembrane Mucin 1 (MUC1) | 1q21 | 3 44.8 cM |
| AF051102 | Ggh | NM 003878 gamma-glutamyl hydrolase (conjugase, folylpolygamma-glutamyl hydrolase) (GGH) | 8q12.3 | 4 A3-A5 |
| U22516 | Ang | M11567 angiogenin | 14q11.1-q11.2 | 14 18.0 cM |
| AW122851 | 1110002023Rik; Fkbp11 similar; FK506 binding protein 11 | NM 016594 FK506 binding protein 11 (FKBP11) | 12q13.12 | 15 F2 |
| Al852985 | 1810014L12Rik; MAC30 hypothetical protein | AU150186; NM 014573 NT2RP2 | 17q11.2 | 11 45.19 cM |
| AV367855 | C1qb | NM 000491 complement component 1, q subcomponent, beta polypeptide (C1QB) | 1p36.3-p34.1 | 466.1 cM |
| AJ222586 | Nucb2 | NM 005013 nucleobindin 2 (NUCB2) | 11p15.1-p14 | 7 F1 |
| M77123 | Vtn | NM 000638 vitronectin (serum spreading factor, somatomedin B,complement S-protein) (VTN) | 17q11 | 11 45.09 cM |

The respective orthologs in different mammalian species can be found with the UNIGENE database or with the Eukaryotic Gene Orthologs (EGO) database: http://www.tigr.org/tdb/tgi/ego/. This is a database for orthologous genes in eukaryotes.

### Example 3: Antibodies against human ortholgs

The following antibodies are directed against human marker proteins of the invention. These antibodies can be used in different detection and measurement methods according to the invention.

| **Probe Set ID** | **Genbank** | **Common gene symbol** | **Common name** | **Antibody** |
|---|---|---|---|---|
| 161890_f_at | AV371861 | Pap | Pancreatic associated protein | Rec Human REG1A: Cat No. RP-10087-P1ABX |
| 94716_f_at | M17962 | Klk9 kallikrein 9 and in human: kallikrein 6, kallikrein 1, nerve growth factor, alpha,RIKEN cDNA 1700127D06 gene | kallikrein 9 | KLK9 (C-15): sc-20385 Santa Cruz |
| 92601_at | AA674409 | Pnliprp1 | Pancreatic lipase related protein 1 | Ahmed Aloulou Protein Expr Purif. 2006 Jan 31; |
| 100061_f_at | M13500 | Klk6; Kal; Klk1; mGk-6; 0610007D04Rik | kallikrein 6 | KLK6 (N-16): sc-20376 Santa Cruz |
| 93302_at | U78770 | Tff2; SP; mSP | Trefoil factor 2 spasmolytic protein 1 | SP (P-19): sc-23558 Santa Cruz |
| 92252_at | D85605 | Cckar; AW106902 | Cholecystokinin receptor | CCK-AR (G-17): sc-16173 Santa Cruz |
| 104495_f_at | Y00500 | Klk5; mGK-5 | Kallikrein 5 | KLK5 (N-14): sc-20375 Santa Cruz |
| 160132_at | AA710635 | Clps | Colipase pancreatic | Josiane De Caro et al Biochim Biophys Acta. 2004 Sep 1;1701(1-2):89-99. |
| 160070_at | M30687 | Pnliprp2 | pancreatic lipase related protein 2 | Ahmed Aloulou Protein Expr Purif. 2006 Jan 31 |
| 160213_at | D14010 | Reg1 | Islet Regenerating | Rec Human REG1A: Cat No. RP-10087-P1ABX |
| 101058_at | J00356 | Amy1 | Amylase 1 | Amylase (G-10): sc-46657 |
| 97519_at | X13986 | Spp1 | secreted phosphoprotein 1 | Rabit polyclonal RB-9097 |
| 160120_i_at | AI327450 | Pla2g1b | Phospholipase A2, group 1B | Brant K et al Biol Reprod. 2006 Jan 25; [Epub ahead of print] |
| 99939_at | U37386 | Cel | Carboxyl ester lipase | Aho HJ et al Int J Pancreatol. 1989 Sep;5(2):123-34. |
| 93783_at | M27347 | Ela1 | | http://www.labvision.com/pdf/1841. pdf |
| 94037_at | X04573 | Ela2 | | Shirasu Y et al Hybridoma. 1988 Oct 7;(5):485-93. |
| 102918_at | M84683 | Muc1 | | CD24 (GPI-linked surface mucin) Ab-1 |
| 93575_at | AF051102 | Ggh | | Daniel I. R. Spencer et al Proteomics Volume 2, Issue 3 , Pages 271 - 279 |
| 94392_f_at | U22516 | Ang | | Ahn EH et al Proteomics. 2006 Feb;6(4):1104-9 |
| 162276_i_at | AV367855 | C1qb | | Ogden C, Elkon K Curr Dir Autoimmun. 2006;9:120-42 |
| 102197_at | AJ222586 | Nucb2 | | Kawano J et al Histochem Cell Biol. 2001 May;115(5):421-8 |
| 98549_at | M77123 | Vtn | | Kamikubo Y et al Biochemistry. 2006 Mar 14;45(10):3297-306 |

### Example 4: determination of plasma level of a marker protein.

In this example, the marker protein chosen is Osteopontin (OPN); the same protocol may however easily be adapted in order to determine the levels of another marker protein of the invention in the peripheral blood.

Opn levels in plasma can be measured using a solid-phase sandwich enzyme-linked immunosorbant assay kit (Immuno-Biological Laboratory, Gumma, Japan). In a published report {Schorge 2004}, the methodology suggested is as follows:

Microplates were first pre coated with antihuman OPN rabbit lgG [100 µl of 20 µg/ml in 0.1 M carbonate buffer (pH, 9.5)] and blocked with 1% BSA and 0.05% Tween 20. Plasma and standard OPN samples were diluted with 1% BSA and 0.05% Tween 20 in PBS and incubated for 1 h at 37°C. After seven washes with 0.05% Tween 20 in phosphate buffer, horseradish peroxidase-labeled conjugated antihuman OPN (10A16) mouse monoclonal antibody (100 µl of 2 ng/ml) was added and incubated for 30 min at 4°C. After nine washes, 100 µl of tetramethyl benzidine buffer was added, and the signal was allowed to develop for 30 min at room temperature. The reaction was stopped with 100µl of 1 N sulfuric acid. The absorbance at 450 nm was measured by an automatic ELISA reader (Bio-Rad, Hercules, CA). Results were converted from the mean absorbance of duplicate wells after subtraction of background values. Recombinant human OPN protein (IBL) was used as a standard.

The standard curve was prepared simultaneously with the measurement of test samples. Reagent blank, test-sample blank and internal controls of plasma samples were used to normalize OPN values obtained from each experiment.

A modified protocol has been also proposed in another publication {Koopmann, 2004}.

### Example 5: detection of autoantibodies directed against a marker protein.

In this example, the marker protein chosen is Osteopontin (OPN); the same protocol may however easily be adapted to other marker proteins of the invention.

Autoantibodies for OPN have been found in the serum of patents with osteoarthritis and rheumatoid arthritis. ELISA and Western blotting can be used for OPN measurements. Native purified human OPN (nhOPN) exist and can be used as control and for the establishment of standard Curves {Sakata, 2001}.

### References:

Andre, I., Gonzalez, A., Wang, B., Katz, J., Benoist, C. and Mathis, D. (1996) Checkpoints in the progression of autoimmune disease: lessons from diabetes models. Proc Natl Acad Sci U S A, 93, 2260-2263.
Ashkar, S., et al (2000) Eta-1 (osteopontin): an early component of type-1 (cell-mediated) immunity. Science, 287, 860-864.
Barker, J.M. (2006) Type 1 diabetes associated autoimmunity: Natural History, Genetic Associations and Screening. J Clin Endocrinol Metab.
Baus-Loncar, M., Kayademir, T., Takaishi, S. and Wang, T. (2005) Trefoil factor family 2 deficiency and immune response. Cell Mol Life Sci, 62, 2947-2955.
Bines, J.E., Truby, H.D., Armstrong, D.S., Carzino, R. and Grimwood, K. (2005) Vitamin A and E deficiency and lung disease in infants with cystic fibrosis. J Paediatr Child Health, 41, 663-668.
Bond, M.D. and Vallee, B.L. (1990) Isolation and sequencing of mouse angiogenin DNA. Biochem Biophys Res Commun, 171, 988-995.
Bottazzo, G.F. (1986) Lawrence lecture. Death of a beta cell: homicide or suicide? Diabet Med, 3, 119-130.
Buchler, P., Gazdhar, A., Schubert, M., Giese, N., Reber, H.A., Hines, O.J., Giese, T., Ceyhan, G.O., Muller, M., Buchler, M.W. and Friess, H. (2005) The Notch signaling pathway is related to neurovascular progression of pancreatic cancer. Ann Surg, 242, 791-800, discussion 800-791.
Chabas, D., et al. (2001) The influence of the proinflammatory cytokine, osteopontin, on autoimmune demyelinating disease. Science, 294, 1731-1735.
Chen, C.Y., Lin, X.Z., Wu, H.C. and Shiesh, S.C. (2005) The value of biliary amylase and Hepatocarcinoma-Intestine-Pancreas/Pancreatitis-associated Protein I (HIP/PAP-I) in diagnosing biliary malignancies. Clin Biochem, 38, 520-525.
Cheng, Z.J., Harikumar, K.G., Ding, W.Q., Holicky, E.L. and Miller, L.J. (2005) Analysis of the cellular and molecular mechanisms of trophic action of a misspliced form of the type B cholecystokinin receptor present in colon and pancreatic cancer. Cancer Lett, 222, 95-105.
Cristillo, A.D. and Bierer, B.E. (2002) Identification of novel targets of immunosuppressive agents by cDNA-based microarray analysis. J Biol Chem, 277, 4465-4476.
de Vos, S., et al. (2003) Gene expression profile of serial samples of transformed B-cell lymphomas. Lab Invest, 83, 271-285.
Dhar, D.K., et al. (2005) Expression of trefoil factor family members correlates with patient prognosis and neoangiogenesis. Clin Cancer Res, 11, 6472-6478.
Esaki, T., Roy, K., Yao, R., Galivan, J. and Sirotnak, F.M. (1998) Cloning of mouse gamma-glutamyl hydrolase in the form of two cDNA variants with different 5' ends and encoding alternate leader peptide sequences. Gene, 219, 37-44.
Escandell, J.M., et al. (2006) Dihydrocucurbitacin B, isolated from Cayaponia tayuya, reduces damage in adjuvant-induced arthritis. Eur J Pharmacol.
Graf, R., et al. (2005) Exocrine Meets Endocrine: Pancreatic Stone Protein and Regenerating Protein-Two Sides of the Same Coin. J Surg Res.
Greenbaum, D., et al. (2001) Interrelating different types of genomic data, from proteome to secretome: 'oming in on function. Genome Res, 11, 1463-1468.
Grusby, M.J., et al. (1990) Cloning of an interleukin-4 inducible gene from cytotoxic T lymphocytes and its identification as a lipase. Cell, 60, 451-459.
He, P., et al. (2004) Identification of carboxypeptidase E and gamma-glutamyl hydrolase as biomarkers for pulmonary neuroendocrine tumors by cDNA microarray. Hum Pathol, 35, 1196-1209.
Hochberg, Y. & Benjamini Y. (1990). More powerful procedures for multiple significance testing. Stat. Med. 9: 811-8.
Imamura, T., et al. (2002) Protection from pancreatitis by the zymogen granule membrane protein integral membrane-associated protein-1. J Biol Chem, 277, 50725-50733.
Jamal, A.M., et al. (2005) Morphogenetic plasticity of adult human pancreatic islets of Langerhans. Cell Death Differ, 12, 702-712.
Koopman, J., et al. (2004) Evaluation of Osteopontin as Biomarker for pancreatic adenocarcinoma. Cancer Epidemiol Biomarkers Prev, 13(3), 487-491.
Leiter, E.H. (1993) The NOD mouse: A model for analyzing the interplay between heredity and environment in development of autoimmune disease. ILAR News, Vol. 35, p. 4.
Li, H., et al. (2004). Analysis of oligonucleotide array experiments with repeated measures using mixed models. BMC Bioinformatics. 5: 209.
Lindner, I., et al. (2005) Putative association between a new polymorphism in exon 3 (Arg109Cys) of the pancreatic colipase gene and type 2 diabetes mellitus in two independent Caucasian study populations. Mol Nutr Food Res, 49, 972-976.
Melanitou, E. (2005) The autoimmune contrivance: genetics in the mouse model. Clin Immunol, 117, 195-206.
Melanitou, E. (2005) Functional genomics in early autoimmunity. Ann N Y Acad Sci, 1050, 64-72.
Melanitou, E., Devendra, D., Liu, E., Miao, D. and Eisenbarth, G.S. (2004) Early and quantal (by litter) expression of insulin autoantibodies in the nonobese diabetic mice predict early diabetes onset. J Immunol, 173, 6603-6610.
Natasha, T., Kuhn, M., Kelly, O. and Rittling, S.R. (2006) Override of the osteoclast defect in osteopontin-deficient mice by metastatic tumor growth in the bone. Am J Pathol, 168, 551-561.
Paradis, V., et al. (2005) Identification of a new marker of hepatocellular carcinoma by serum protein profiling of patients with chronic liver diseases. Hepatology, 41, 40-47.
Patarca, R., et al. (1993) Molecular and cellular basis of genetic resistance to bacterial infection: the role of the early T-lymphocyte activation-1/osteopontin gene. Crit Rev Immunol, 13, 225-246.
Pavel, M.E., et al. (2005) Circulating levels of angiogenic cytokines can predict tumour progression and prognosis in neuroendocrine carcinomas. Clin Endocrinol (Oxf), 62, 434-443.
Pinelli, M., Bindi, M., Rosada, J., Scatena, P. and Castiglioni, M. (2006) Amylase: A disease activity index in multiple myeloma? Leuk Lymphoma, 47, 151-154.
Politou, M., Naresh, K., Terpos, E., Crawley, D., Lampert, I., Apperley, J.F. and Rahemtulla, A. (2005) Anti-angiogenic effect of bortezomib in patients with multiple myeloma. Acta Haematol, 114, 170-173.
Price, R.D., et al. (2005) FK1706, a novel non-immunosuppressive immunophilin: neurotrophic activity and mechanism of action. Eur J Pharmacol, 509, 11-19.
Raeder, H., et al. (2006) Mutations in the CEL VNTR cause a syndrome of diabetes and pancreatic exocrine dysfunction. Nat Genet, 38, 54-62.
Reimund, J.M., et al. (2005) Factors contributing to infectious diarrhea-associated pancreatic enzyme alterations. Gastroenterol Clin Biol, 29, 247-253.
Ridgway, W.M., Fasso, M., Lanctot, A., Garvey, C. and Fathman, C.G. (1996) Breaking self-tolerance in nonobese diabetic mice. J Exp Med, 183, 1657-1662.
Sakata M et al. (2004). J Rheumatol.; 28(7):1492-1495.
Schorge J.O. et al. (2004). Clinical Cancer Research., 10, 3474-3478.
Seiffert, D., Keeton, M., Eguchi, Y., Sawdey, M. and Loskutoff, D.J. (1991) Detection of vitronectin mRNA in tissues and cells of the mouse. Proc Natl Acad Sci U S A, 88, 9402-9406.
Silva, F., et al. (2001) MUC1 gene polymorphism in the gastric carcinogenesis pathway. Eur J Hum Genet, 9, 548-552.
Snyder, D.W., et al. (1999) Pharmacology of LY315920/S-5920, [[3-(aminooxoacetyl)-2-ethyl-1-(phenylmethyl)-1H-indol-4-yl]oxy] acetate, a potent and selective secretory phospholipase A2 inhibitor: A new class of anti-inflammatory drugs, SPI. J Pharmacol Exp Ther, 288, 1117-1124.
Stamey, T.A., Yang, N., Hay, A.R., McNeal, J.E., Freiha, F.S. and Redwine, E. (1987) Prostate-specific antigen as a serum marker for adenocarcinoma of the prostate. N Engl J Med, 317, 909-916.
Takata, Y., Takiguchi, S., Kataoka, K., Funakoshi, A., Miyasaka, K. and Kono, A. (1997) Mouse cholecystokinin type-A receptor gene and its structural analysis. Gene, 187, 267-271.
Thim, L. and May, F.E. (2005) Structure of mammalian trefoil factors and functional insights. Cell Mol Life Sci, 62, 2956-2973.
Tomasetto, C., et al. (1990) hSP, the domain-duplicated homolog of pS2 protein, is co-expressed with pS2 in stomach but not in breast carcinoma. Embo J, 9, 407-414.
Wasle, B., Turvey, M., Larina, O., Thorn, P., Skepper, J., Morton, A.J. and Edwardson, J.M. (2005) Syncollin is required for efficient zymogen granule exocytosis. Biochem J, 385, 721-727.
Yamaguchi, Y., et al. (2002) Identification of multiple novel epididymis-specific beta-defensin isoforms in humans and mice. J lmmunol, 169, 2516-2523.
Yamamoto, N., et al. (2003) Essential role of the cryptic epitope SLAYGLR within osteopontin in a murine model of rheumatoid arthritis. J Clin Invest, 112, 181-188.
Yamasaki, N., Sugimura, K., Hiida, M., Naito, T. and Watanabe, T. (1987) Sequence analysis of a cDNA clone of a gene encoding a component of a putative phosphorylcholine-specific T suppressor factor and functional property of its gene product. Eur J Immunol, 17, 247-253.
Yousef, G.M. and Diamandis, E.P. (2000) The expanded human kallikrein gene family: locus characterization and molecular cloning of a new member, KLK-L3 (KLK9). Genomics, 65, 184-194.
Yuan, R.H., Jeng, Y.M., Chen, H.L., Hsieh, F.J., Yang, C.Y., Lee, P.H. and Hsu, H.C. (2005) Opposite roles of human pancreatitis-associated protein and REG1A expression in hepatocellular carcinoma: association of pancreatitis-associated protein expression with low-stage hepatocellular carcinoma, beta-catenin mutation, and favorable prognosis. Clin Cancer Res, 11, 2568-2575.

## Claims

1. Method for the early diagnosis of a disease having a pre-inflammatory phase and/or of a disease-prone state, in a mammal, prior to any clinical signs, comprising
a) measuring the level of at least a marker protein chosen amongst the following murine proteins Pap, Reg3a, Reg2, Cel, Reg1, Tff2, Clps, Spp1, Klk9, Klk6, Rib1, Klk5, Muc1, Cckar, Ggh, Ang, Nucb2, Pnliprp2, Pla2g1b, Ela1, Ela2, 2210010C04Rik, Pnliprp1, Itmap1, Vtn, C1qb, Sycn, Amy1, Ctrb1, 1110002023Rik, 1810014L12Rik and their mammalian orthologs, in a body fluid or tissue sample obtained from said mammal,
b) comparing the measured level to a reference level for said marker protein and
c) diagnosing the later onset of said disease or diagnosing a disease-prone state if the measured level is significantly superior to the reference level.

2. The method according to claim 1, wherein said protein is chosen amongst the following murine proteins Pap, Reg3a, Reg2, Cel, Reg1, Tff2, Clps and Spp1, and their respective mammalian orthologs, and wherein said protein is implicated in tissue regeneration or integrity.

3. The method according to claim 1, wherein said protein is chosen amongst the following murine proteins Klk9, Klk6, Rib1, Klk5, Muc1, Cckar, Ggh, Ang, Nucb2 and Spp1, and their respective mammalian orthologs and wherein said protein is implicated in tumorigenesis.

4. The method according to claim 1, wherein said protein is chosen amongst the following murine proteins Pnliprp2, Pla2g1b, Ela1, Ela2, 2210010C04Rik, Pnliprp1, Itmap1, Vtn, C1qb and Spp1, and their respective mammalian orthologs and wherein said protein has an immune function.

5. The method according to claim 1, wherein said protein is chosen amongst the following murine proteins Sycn, Amy1, Ctrb1, 1110002O23Rik and 1810014L12Rik, and their respective mammalian orthologs.

6. The method according to claim 4, wherein said protein is elastase 1 or a mammalian ortholog.

7. The method according to any one of claims 1 to 6, wherein said body fluid is selected from blood, serum, plasma, urine, saliva, sweat and synovial fluid.

8. The method according to any one of claims 1 to 7, wherein said mammal is a human being and the protein is chosen amongst the following proteins: Regenerating islet-derived 3 alpha (REG3A), Pancreatitis associated protein 1 (PAP), Regenerating protein I beta, prostrate Kallikrein 2 (KLK2), Syncollin (SYCN), Pancreatic lipase-related protein 1 (PNUPRP1), Kallikrein6 (KLK6), pancreatic Ribonuclease, RNase A family, 1 (RNASE1), Spasmolytic polypeptide (SP), Cholecystokinin A receptor, CUB and zona pellucida-like domains 1 (CUZD1 or ERG-1), pancreatic Colipase, (CLPS), pancreatic lipase-related protein 2, Lithostathine (REG1A), Amylase 1, Osteopontin, Phospholipase A2, group IB (PLA2G1B), Carboxyl ester lipase (bile salt-stimulated lipase) (CEL), Elastase 1 precursor (ELA1), Chymotrypsinogen B1 (CTRB1), Pancreatic elastase IIA, Protease, serine, 3 (mesotrypsin) (PRSS3), transmembrane Mucin 1 (MUC1), gamma-glutamyl hydrolase (conjugase, folylpolygammaglutamyl hydrolase) (GGH), Angiogenin, FK506 binding protein 11 (FKBP11), NT2RP2, Complement component 1, q subcomponent, beta polypeptide (C1QB), Nucleobindin 2 (NUCB2) and Vitronectin (serum spreading factor, somatomedin B,complement S-protein) (VTN).

9. The method according to claim 8, wherein the protein is chosen from the group comprising Regenerating islet-derived 3 alpha (REG3A), Pancreatitis associated protein 1 (PAP), Regenerating protein I beta, Spasmolytic polypeptide (SP), pancreatic Colipase, (CLPS), Lithostathine (REG1A), Carboxyl ester lipase (bile salt-stimulated lipase) (CEL) and Osteopontin, and wherein said protein is implicated in tissue regeneration or integrity.

10. The method according to claim 8, wherein the protein is chosen from the group comprising prostrate Kallikrein 2 (KLK2), Kallikrein6 (KLK6), pancreatic Ribonuclease, RNase A family, 1 (RNASE1), Cholecystokinin A receptor, transmembrane Mucin 1 (MUC1), gamma-glutamyl hydrolase (conjugase, folylpolygammaglutamyl hydrolase) (GGH), Angiogenin, Nucleobindin 2 (NUCB2) and Osteopontin, and wherein said protein is implicated in tumorigenesis.

11. The method according to claim 8, wherein the protein is chosen from the group comprising Pancreatic lipase-related protein 1 (PNLIPRP1), CUB and zona pellucida-like domains 1 (CUZD1 or ERG-1), pancreatic lipase-related protein 2, Phospholipase A2, group IB (PLA2G1B), Elastase 1 precursor (ELA1), Pancreatic elastase IIA, Protease, serine, 3 (mesotrypsin) (PRSS3), Complement component 1, q subcomponent, beta polypeptide (C1QB), Vitronectin (serum spreading factor, somatomedin B,complement S-protein) (VTN) and Osteopontin, and wherein said protein has an immune function.

12. The method according to claim 8, wherein the protein is chosen from the group comprising Syncollin (SYCN), Amylase 1, Chymotrypsinogen B1 (CTRB1), FK506 binding protein 11 (FKBP11) and NT2RP2.

13. The method according to any one of claims 1 to 12, wherein said disease having a pre-inflammaroty phase is an autoimmune disease.

14. The method according to claim 13, wherein said autoimmune disease is type 1 diabetes mellitus, multiple sclerosis, rheumatoid arthritis, collagen induced arthritis or autoimmune hepatitis.

15. The method according to any one of claims 1 to 13, wherein said autoimmune disease is type 1 diabetes mellitus and the diagnosis is made before any clinical sign of insulitis.

16. The method according to any one of claims 1 to 15, wherein the method also comprises the detection in the mammal to be diagnosed of the presence of insulin auto-antibody.

17. The method according to any one of claims 1 to 15, wherein the mammal to be diagnosed presents insulin auto-antibody.

18. The method according to any one of claims 1 to 17, wherein said mammal is a human less than 2 years.

19. The method according to any one of claims 1 to 12, wherein said disease is a cancer.

20. The method according to any one of claims 1 to 12, wherein said disease is an infection.

21. The method according to any one of claims 1 to 20, wherein steps a) to b) are repeated twice, simultaneously or sequentially, with two different marker proteins and wherein the later onset of said disease, or disease-prone state, is diagnosed only if the measured levels for both marker proteins are significantly superior to the reference levels.

22. The method according to any one of claims 1 to 20, wherein steps a) to b) are repeated at least 5 times, simultaneously or sequentially, with at least 5 different marker proteins and wherein the later onset of said disease is diagnosed only if the measured levels for at least two of the marker proteins are significantly superior to the reference levels.

23. The method according to any one of claims 1 to 22, wherein said level is measured with a labelled ligand binding specifically the chosen protein, preferably a monoclonal antibody.

24. Use of a protein chosen amongst the following murine proteins Pap, Reg3a, Reg2, Cel, Reg1, Tff2, Clps, Spp1, Klk9, Klk6, Rib1, Klk5, Muc1, Cckar, Ggh, Ang, Nucb2, Pnliprp2, Pla2g1b, Ela1, Ela2, 2210010C04Rik, Pnliprp1, Itmap1, Vtn, C1qb, Sycn, Amy1, Ctrb1, 1110002O23Rik and 1810014L12Rik, and their mammalian orthologs, as a seric marker for the predisposition to a disease having a pre-inflammatory phase or for the early pre-inflammatory condition in autoimmune diseases, before any clinical signs in a mammal.

25. Use according to claim 24, wherein said protein is chosen amongst the following murine proteins Pap, Reg3a, Reg2, Cel, Reg1, Tff2, Clps and Spp1, and their respective mammalian orthologs and wherein said protein is implicated in tissue regeneration or integrity.

26. Use according to claim 24, wherein said protein is chosen amongst the following murine proteins Klk9, Klk6, Rib1, Klk5, Muc1, Cckar, Ggh, Ang, Nucb2 and Spp1, and their respective mammalian orthologs, and wherein said protein is implicated in tumorigenesis.

27. Use according to claim 24, wherein said protein is chosen amongst the following murine proteins Pnliprp2, Pla2g1b, Ela1, Ela2, 2210010C04Rik, Pnliprp1, Itmap1, Vtn, C1qb and Spp1, and their respective mammalian orthologs, and wherein said protein has an immune function.

28. Use according to claim 24, wherein said protein is chosen amongst the following murine proteins Sycn, Amy1, Ctrb1, 1110002023Rik and 1810014L12Rik, and their respective mammalian orthologs.

29. Use according to claim 27, wherein said protein is elastase 1 or its mammalian ortholog.

30. Use according to claim 24, wherein said mammal is a human being and the protein is to be chosen from the group comprising: Regenerating islet-derived 3 alpha (REG3A), Pancreatitis associated protein 1 (PAP), Regenerating protein I beta, prostrate Kallikrein 2 (KLK2), Syncollin (SYCN), Pancreatic lipase-related protein 1 (PNLIPRP1), Kallikrein6 (KLK6), pancreatic Ribonuclease, RNase A family, 1 (RNASE1), Spasmolytic polypeptide (SP), Cholecystokinin A receptor, CUB and zona pellucida-like domains 1 (CUZD1 or ERG-1), pancreatic Colipase, (CLPS), pancreatic lipase-related protein 2, Lithostathine (REG1A), Amylase 1, Osteopontin, Phospholipase A2, group IB (PLA2G1B), Carboxyl ester lipase (bile salt-stimulated lipase) (CEL), Elastase 1 precursor (ELA1), Chymotrypsinogen B1 (CTRB1), Pancreatic elastase IIA, Protease, serine, 3 (mesotrypsin) (PRSS3), transmembrane Mucin 1 (MUC1), gamma-glutamyl hydrolase (conjugase, folylpolygammaglutamyl hydrolase) (GGH), Angiogenin, FK506 binding protein 11 (FKBP11), NT2RP2, Complement component 1, q subcomponent, beta polypeptide (C1QB), Nucleobindin 2 (NUCB2) and Vitronectin (serum spreading factor, somatomedin B, complement S-protein) (VTN).

31. Use according to claim 30, wherein the protein is chosen from the group comprising Regenerating islet-derived 3 alpha (REG3A), Pancreatitis associated protein 1 (PAP), Regenerating protein I beta, Spasmolytic polypeptide (SP), pancreatic Colipase, (CLPS), Lithostathine (REG1A), Carboxyl ester lipase (bile salt-stimulated lipase) (CEL) and Osteopontin, and wherein said protein is implicated in tissue regeneration or integrity.

32. Use according to claim 30, wherein the protein is chosen from the group comprising prostrate Kallikrein 2 (KLK2), Kallikrein6 (KLK6), pancreatic Ribonuclease, RNase A family, 1 (RNASE1), Cholecystokinin A receptor, transmembrane Mucin 1 (MUC1), gamma-glutamyl hydrolase (conjugase, folylpolygammaglutamyl hydrolase) (GGH), Angiogenin, Nucleobindin 2 (NUCB2) and Osteopontin, and wherein said protein is implicated in tumorigenesis.

33. Use according to claim 30, wherein the protein is chosen from the group comprising Pancreatic lipase-related protein 1 (PNLIPRP1), CUB and zona pellucida-like domains 1 (CUZD1 or ERG-1), pancreatic lipase-related protein 2, Phospholipase A2, group IB (PLA2G1B), Elastase 1 precursor (ELA1), Pancreatic elastase IIA, Protease, serine, 3 (mesotrypsin) (PRSS3), Complement component 1, q subcomponent, beta polypeptide (C1QB), Vitronectin (serum spreading factor, somatomedin B,complement S-protein) (VTN) and Osteopontin, and wherein said protein has an immune function.

34. Use according to claim 30, wherein the protein is chosen from the group comprising Syncollin (SYCN), Amylase 1, Chymotrypsinogen B1 (CTRB1), FK506 binding protein 11 (FKBP11) and NT2RP2.

35. Use according to any one of claims 24 to 34, wherein said disease is an autoimmune disease, preferably type 1 diabetes.

36. Use according to any one of claims 24 to 34, wherein said mammal is a human having less than 2 years.

37. Use of a ligand specifically binding a protein chosen from the group comprising: Regenerating islet-derived 3 alpha (REG3A), Pancreatitis associated protein 1 (PAP), Regenerating protein I beta, prostrate Kallikrein 2 (KLK2), Syncollin (SYCN), Pancreatic lipase-related protein 1 (PNLIPRP1), Kallikrein6 (KLK6), pancreatic Ribonuclease, RNase A family, 1 (RNASE1), Spasmolytic polypeptide (SP), Cholecystokinin A receptor, CUB and zona pellucida-like domains 1 (CUZD1 or ERG-1), pancreatic Colipase, (CLPS), pancreatic lipase-related protein 2, Lithostathine (REG1A), Amylase 1, Osteopontin, Phospholipase A2, group IB (PLA2G1B), Carboxyl ester lipase (bile salt-stimulated lipase) (CEL), Elastase 1 precursor (ELA1), Chymotrypsinogen B1 (CTRB1), Pancreatic elastase IIA, Protease, serine, 3 (mesotrypsin) (PRSS3), transmembrane Mucin 1 (MUC1), gamma-glutamyl hydrolase (conjugase, folylpolygammaglutamyl hydrolase) (GGH), Angiogenin, FK506 binding protein 11 (FKBP11), NT2RP2, Complement component 1, q subcomponent, beta polypeptide (C1QB), Nucleobindin 2 (NUCB2) and Vitronectin (serum spreading factor, somatomedin B, complement S-protein) (VTN) in a method for diagnosing, before any clinical signs, a disease or a predisposition to a disease having a pre-inflammatory phase.

38. Use according to claim 37 wherein said ligand is an antibody.

39. Use according to claim 37 or 38, wherein said disease is an autoimmune disease, preferably type 1 diabetes.

40. Use of an agent capable of modulating the activity of a protein chosen from the group comprising: Regenerating islet-derived 3 alpha (REG3A), Pancreatitis associated protein 1 (PAP), Regenerating protein I beta, Syncollin (SYCN), Pancreatic lipase-related protein 1 (PNLIPRP1), pancreatic Ribonuclease, RNase A family, 1 (RNASE1), Spasmolytic polypeptide (SP), Cholecystokinin A receptor, CUB and zona pellucida-like domains 1 (CUZD1 or ERG-1), pancreatic Colipase, (CLPS), pancreatic lipase-related protein 2, Lithostathine (REG1A), Amylase 1, Osteopontin, Phospholipase A2, group IB (PLA2G1B), Carboxyl ester lipase (bile salt-stimulated lipase) (CEL), Elastase 1 precursor (ELA1), Chymotrypsinogen B1 (CTRB1), Pancreatic elastase IIA, Protease, serine, 3 (mesotrypsin) (PRSS3), transmembrane Mucin 1 (MUC1), gamma-glutamyl hydrolase (conjugase, folylpolygammaglutamyl hydrolase) (GGH), Angiogenin, FK506 binding protein 11 (FKBP11), NT2RP2, Complement component 1, q subcomponent, beta polypeptide (C1QB), Nucleobindin 2 (NUCB2) and Vitronectin (serum spreading factor, somatomedin B complement S-protein) (VTN), for the manufacture of a medicament for treating patients suffering from an autoimmune disease, preferably from type 1 diabetes.

41. Use of an agent capable of modulating the activity of a protein chosen from the group comprising: Regenerating islet-derived 3 alpha (REG3A), Pancreatitis associated protein 1 (PAP), Regenerating protein I beta, Syncollin (SYCN), Pancreatic lipase-related protein 1 (PNLIPRP1), pancreatic Ribonuclease, RNase A family, 1 (RNASE1), Spasmolytic polypeptide (SP), Cholecystokinin A receptor, CUB and zona pellucida-like domains 1 (CUZD1 or ERG-1), pancreatic Colipase, (CLPS), pancreatic lipase-related protein 2, Lithostathine (REG1A), Amylase 1, Osteopontin, Phospholipase A2, group IB (PLA2G1B), Carboxyl ester lipase (bile salt-stimulated lipase) (CEL), Elastase 1 precursor (ELA1), Chymotrypsinogen B1 (CTRB1), Pancreatic elastase IIA, Protease, serine, 3 (mesotrypsin) (PRSS3), transmembrane Mucin 1 (MUC1), gamma-glutamyl hydrolase (conjugase, folylpolygammaglutamyl hydrolase) (GGH), Angiogenin, FK506 binding protein 11 (FKBP11), NT2RP2, Complement component 1, q subcomponent, beta polypeptide (C1QB), Nucleobindin 2 (NUCB2) and Vitronectin (serum spreading factor, somatomedin B,complement S-protein) (VTN), for the manufacture of a medicament for treating patients, being disease-prone for an autoimmune disease, preferably for type 1 diabetes, before clinical signs.

42. Use according to any one of claims 40 and 41, wherein said treated patient has no sign of insulitis.

43. Use according to any one of claims 40 to 42, wherein said agent decreases the activity of said chosen protein.

44. Use according to any one of claims 40 to 43, wherein said agent is an antibody, preferably a monoclonal antibody.

45. Kit for diagnosing a predisposition to a disease having a pre-inflammatory phase, or for diagnosing said disease, before any clinical signs, comprising:
- a means for dosing a protein chosen from the group comprising: Regenerating islet-derived 3 alpha (REG3A), Pancreatitis associated protein 1 (PAP), Regenerating protein I beta, prostrate kallikrein 2 (KLK2), Syncollin (SYCN), Pancreatic lipase-related protein 1 (PNUPRP1), Kallikrein6 (KLK6), pancreatic Ribonuclease, RNase A family, 1 (RNASE1), Spasmolytic polypeptide (SP), Cholecystokinin A receptor, CUB and zona pellucida-like domains 1 (CUZD1 or ERG-1), pancreatic Colipase, (CLPS), pancreatic lipase-related protein 2, Lithostathine (REG1A), Amylase 1, Osteopontin, Phospholipase A2, group IB (PLA2G1B), Carboxyl ester lipase (bile salt-stimulated lipase) (CEL), Elastase 1 precursor (ELA1), Chymotrypsinogen B1 (CTRB1), Pancreatic elastase IIA, Protease, serine, 3 (mesotrypsin) (PRSS3), transmembrane Mucin 1 (MUC1), gamma-glutamyl hydrolase (conjugase, folylpolygammaglutamyl hydrolase) (GGH), Angiogenin, FK506 binding protein 11 (FKBP11), NT2RP2, Complement component 1, q subcomponent, beta polypeptide (C1QB), Nucleobindin 2 (NUCB2) and Vitronectin (serum spreading factor, somatomedin B, complement S-protein) (VTN) and
- a reference level for said protein.

46. Kit according to claim 45, wherein said means for dosing a protein is an antibody, preferably a monoclonal antibody.
